# EUROPEAN PATENT SPECIFICATION

(11) **EP 4 402 478 B1**
(45) Date of publication and mention of the grant of the patent: **28.10.2026**
(21) Application number: 22789503.4
(22) Date of filing: 14.09.2022
(51) Int. Cl.: G01N 33/68, G01N 33/543

(54) **METHOD FOR DETERMINING AT LEAST ONE ANALYTE OF INTEREST**
VERFAHREN ZUR BESTIMMUNG VON MINDESTENS EINEM ANALYTEN VON INTERESSE
PROCÉDÉ DE DÉTERMINATION D'AU MOINS UNE SUBSTANCE À ANALYSER

(30) Priority: 17.09.2021 EP 21197383
(43) Date of publication of application: 24.07.2024
(73) Proprietor: F. Hoffmann-La Roche AG, 4070 Basel (CH); Roche Diagnostics GmbH, 68305 Mannheim (DE)
(72) Inventor: REMPT, Martin, 82377 Penzberg (DE); SEITZ, Manuel Josef, 82377 Penzberg (DE); ZUTH, Christoph, 82377 Penzberg (DE)
(74) Representative: Epping, Claudia Viktoria
(86) International application number: PCT/EP2022/075510
(87) International publication number: WO 2023/041571

(56) References cited:
- WO-A1-2016/174525
- US-A1- 2002 192 676
- US-A1- 2007 054 407
- G. SCHLOSSER ET AL: "Coupling Immunomagnetic Separation on Magnetic Beads with Matrix-Assisted Laser Desorption Ionization-Time of Flight Mass Spectrometry for Detection of Staphylococcal Enterotoxin B", APPLIED AND ENVIRONMENTAL MICROBIOLOGY, vol. 73, no. 21, 1 November 2007 (2007-11-01), US, pages 6945 - 6952, XP055275697, ISSN: 0099-2240, DOI: 10.1128/AEM.01136-07
- MEDHE SHARAD: "Ionization Techniques in Mass Spectrometry: A Review", vol. 04, no. 01, 1 January 2018 (2018-01-01), XP055894336, Retrieved from the Internet <URL:https://www.longdom.org/open-access-pdfs/ionization-techniques-in-mass-spectrometry-a-review-2469-9861-1000126.pdf> DOI: 10.4172/2469-9861.1000126
- SUGIMURA NATSUHIKO ET AL: "Triboionization: a Novel Ionization Method by Peeling of Cohesive Substances for Mass Spectrometry", JOURNAL OF THE AMERICAN SOCIETY FOR MASS SPECTROMETRY, ELSEVIER SCIENCE INC, US, vol. 30, no. 8, 17 May 2019 (2019-05-17), pages 1503 - 1511, XP037128379, ISSN: 1044-0305, [retrieved on 20190517], DOI: 10.1007/S13361-019-02220-8

## Description

### Field of the Invention

The present invention relates to a method for determining at least one analyte of interest and a system automated to perfom the method.

### Background of the Invention

The process of ionization related to matrix assisted ionizations can be laser supported ionization (MALDI/SALDI), matrix supported ionization (MAI) and/or ambient voltage supported ionization. MAI is an ionization method that uses a liquid/solid support media to mix crystalline matrix component(s) e.g. 3-NBN or 2,2'-azobis(2-methylpropane) with the respective analyte and brings it nearby the entrance of a capillary of the mass spectrometer, e.g. heated capillary of the mass spectrometer.

The ionization process occurs by transferring the matrix component together with the analyte into the mass spectrometer entrance.

Schlosser et al. 2007. Coupling Immunomagnetic Separation on Magnetic Beads with Matrix-Assisted Laser Desorption Ionization-Time of Flight Mass Spectrometry for Detection of Staphylococcal Enterotoxin B. Appl Environ Microbiol73, https://doi.org/10.1128/AEM.01136-07 reports that SEB could be detected directly "on beads" by placing the molecular probe on the matrix-assisted laser desorption ionization target plate or, alternatively, "off beads" after its acidic elution.

US 2007/054407 A1 describes methods, compositions, and systems for mass spectrometric analysis of magnetic nanoparticles displaying ligands on their surface. For example, methods of screening a sample for the presence of at least one analyte using ligand conjugated magnetic nanoparticles, magnetic separation, and mass spectrometric analysis. It also relates to MALDI matrix compositions comprising ligand conjugated magnetic nanoparticles.

US 2002/192676 A1 describes a method for determining whether a particular bacteria is present in a liquid mixture of biological material. Reported are the steps of providing a sampling structure comprised of a body to which antibodies for a particular type of bacteria have been bonded and exposing the sampling structure to a liquid medium that may contain whole cell bacteria for the particular type of bacteria.

WO 2016/174525 A1 describes a method for quantitating a target molecule in at least one assay sample comprising: depositing on a biochip at least one first capture molecule; adding at least one target molecule and allowing the target molecule to react with said at least one first capture molecule; adding at least one second capture molecule that reacts with the target molecule, this at least one second capture molecule being attached to at least one nanoparticle or microparticle wherein specific ionizable atoms are part of the at least one nanoparticle or microparticle or at least one second capture molecule being attached to at least one nanoparticle or microparticle comprising specific ionizable atoms being attached at their surface or to said at least one nanoparticle or microparticle having specific ionizable atoms being part of a molecule attached to the at least one nanoparticle or microparticle at their surface or at least one second capture molecule having specific ionizable atoms attached thereto.

Medhe, Sharad. (2018). Ionization Techniques in Mass Spectrometry: A Review. Mass Spectrometry & Purification Techniques. 4. 10.4172/2469-9861.1000126 reports various types of mass ion Sources (Electron impact, Chemical ionization and field ionization, Desorption-Field desorption, Electro spray ionization, matrix assisted desorption ionization, Plasma desorption) which are in use for mass spectroscopy. Ions get affected by change in electrical, magnetic and radiofrequency effect as they containing either positive or negative charges this leads to better resolution of ions.

Sugimura N. et al., Triboionization: a Novel Ionization Method by Peeling of Cohesive Substances for Mass Spectrometry, Journal of the American Society for Mass Spectrometry 2019 30 (8), 1503-1511, DOI: 10.1007/s13361-019-02220-8 reports that a sample compound attached to adhesive tape or plastic wrap was quickly peeled off at a distance of approximately 2 cm from the atmospheric interface of a mass spectrometer. All of the five types of food wrap and 13 types of adhesive tape tested successfully ionized caffeine. Nine out of ten model compounds were detected as the corresponding molecular ions in the positive or negative mode by this ionizing contrivance using an oriented polypropylene adhesive tape. The detected molecular ions were typically protonated molecules or sodium adducts in the positive mode or deprotonated molecules in the negative mode. The elemental compositions of the observed ions were confirmed within 5 ppm by high-resolution mass spectrometry. The triboionization phenomenon was considered to depend on physical and electronic events caused by peeling off a cohesive substance. Triboionization is able to provide a compact ion source using only mechanical mechanisms. Additionally, triboionization allows sticky tape to be used as a convenient sampling device for surface analysis.

However, these methods need are not perfectly compatible with an automated (magnetic) bead handling workflow. The current MAI compounds are limited and therefore more substance classes are desired.

There is thus an urgent need in the art to overcome the above mentioned problems.

It is an object of the present invention to provide a method for determining at least one analyte of interest. The present invention further relates to a sample element, an inlet, a composition, a kit and the use thereof for determining at least one analyte of interest.

This object is or these objects are solved by the subject matter of the independent claims. Further embodiments are subjected to the dependent claims.

### Summary of the Invention

In the following, the present invention relates to the following apects which are covered by the claims. Moreover, examples not being covered by the claims and therefore not being claimed are disclosed to better understand the invention. The term "embodiment" may relate to either.

In an aspect, the present invention relates to a method according to claim 1. Preferred embodiments are covered by the dependent claims 2 to 4.

In an example, the use of the method of one of the embodiments described herein for determining the at least one analyte of interest is mentioned.

In an example, a sample element for determining at least one analyte and suitable to perform the method according to one of the embodiments described herein, comprises
- a substrate surface,
- an ionization matrix arranged on the substrate surface and for using matrix-assisted ionization,
- an analyte-microparticle-complex arranged on the substrate surface,
- wherein the ionization matrix is selected from the group consisting of salsalate, 3-nitrobenzonitrile, 2,2'-azobis(2-methylpropane), 2-nitrobenzonitrile, 5-methyl-2- nitrobenzonitrile, coumarin, methyl-2-methyl-3-nitrobenzoate, methyl-5-nitro-2-furoate, 2-bromo-2-nitropropane-1,3-diol, 3-nitrobenzaldehyde, 6-nitro-o-anisonitrile, phthalic anhydride, or mixtures thereof,
- wherein the ionization matrix and/or analyte-microparticle-complex are crystallized,
- wherein the microparticle of the analyte-microparticle-complex is magnetic,
- wherein the analyte-microparticle-complex and ionization matrix are in contact to each other.

In an example a use of the inlet of one of the embodiments is described herein for determining at least one analyte.

In another aspect, the present invention relates to a system according to claim 5.

In yet another example, a use of the system with inlet of one of the embodiments described herein for determining at least one analyte is described.

In a further example, a composition is described for vacuum or inlet ionization comprising an ionization matrix, wherein the ionization matrix comprises or consists of salsalate.

In yet another example, a use of the composition of the one of the embodiments described herein for determining at least one analyte is described.

In a further example, a kit of one of the embodiments described herein in a method of one of the embodiments described herein or suitable to perform a method of one of the embodiments described herein is described.

In another example, a kit of one of the embodiments described herein in the method of one of the embodiments described herein is described.

### List of Figures

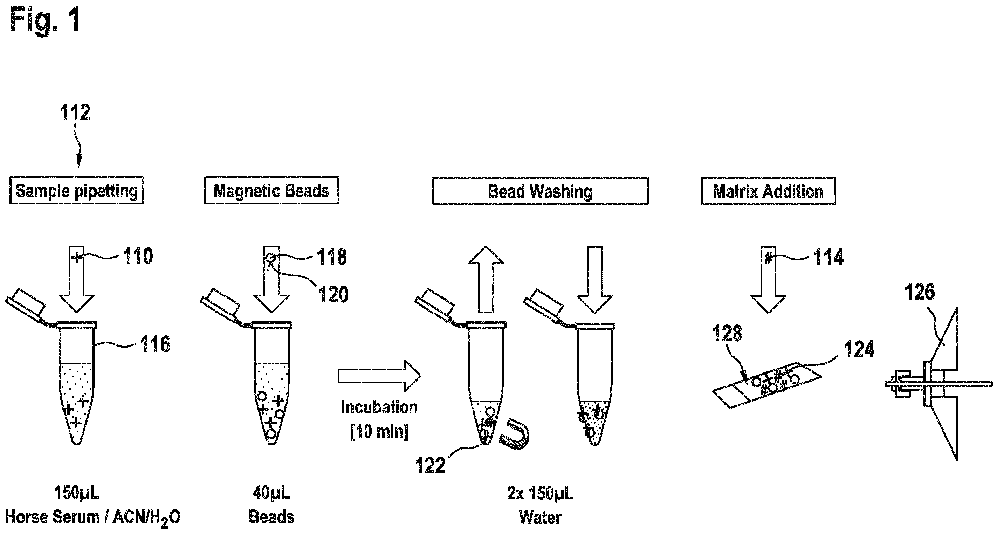

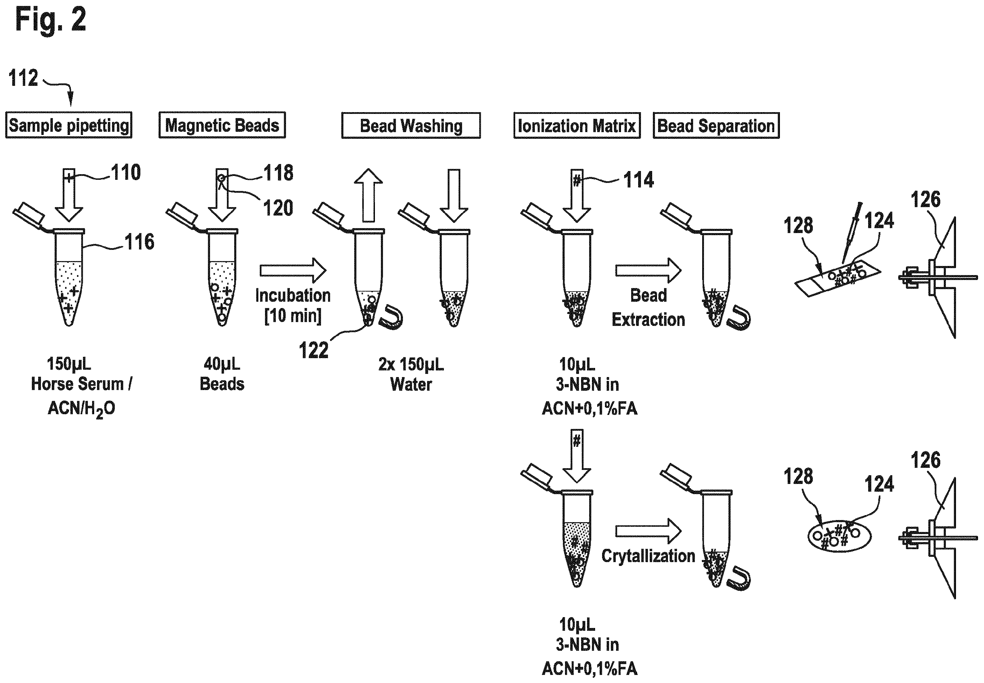

Each of **Figures 1** **and** 2 shows a schematic description of the method for determining at least one analyte in a sample, in particular the matrix ionization microparticle workflow.
**Figures** 3 a) **to** d) show MS spectra (relative abundance vs. time and relative abundance vs. m/z, respectively) of 1 µL residual liquid after magnetic separation spotted on a glass plate.
**Figures** 4 a) **to** d) show MS spectra of 1 µL of a mixture of recrystallized 3-NBN as the ionization matrix and analyte-loaded microparticles as the analyte-microparticle-complex.
**Figure** 5 shows schematic description of the method for determining at least one analyte in a sample, in particular the matrix ionization microparticle workflow.
**Figures** 6 a) **to** d) show MS spectra of analyte-microparticle complex (bead-analyte dispersion) sucked into a triangle-shaped filter with addition of ionization matrix and without ionization matrix.
**Figures** 7 a) and b) show MS spectra of triangle-shaped filter with addition of ionization matrix and missing analyte-microparticle complex (bead-analyte dispersion).
Figure 8 shows schematic descriptions of the method for determining at least one analyte in a sample, in particular the matrix ionization microparticle workflow.
Figures 9 a) to 11 d) show MS spectra of Leucine-Enkephalin coated microparticles with and without pre-crystallized ionization matrix, e.g. 3-NBN matrix.
Figure 12 shows an ionization matrix/analyte ionization of different compounds including the ionization matrix of Salsalate.
Figure 13 shows different nitrobenzene reaction products as MAI alternative.
Figure 14 shows an inlet for ion transport into the mass spectrometer.
Figures 15 **a1)** to d2) show extracted ion mobilograms as well as MS spectra of Leucine-Enkephalin coated microparticles using the inlet for ion transport into the mass spectrometer with and without the use of a filtering material.

### Detailed Description of the Invention

### Definitions

The word "comprise", and variations such as "comprises" and "comprising", will be understood to imply the inclusion of a stated integer or step or group of integers or steps but not the exclusion of any other integer or step or group of integers or steps.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restriction regarding the scope of the invention and without any restriction regarding the possibility of combining the features introduced in such way with other optional or non-optional features of the invention.

As used in this specification and the appended claims, the singular forms "a", "an", and "the" include plural referents, unless the content clearly dictates otherwise.

**Percentages, concentrations, amounts, and other numerical data** may be expressed or presented herein in a "range" format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "4% to 20 %" should be interpreted to include not only the explicitly recited values of 4 % to 20 %, but to also include individual values and sub-ranges within the indicated range. Thus, included in this numerical range are individual values such as 4, 5, 6, 7, 8, 9, 10, ... 18, 19, 20 % and sub-ranges such as from 4-10 %, 5-15 %, 10-20 %, etc. This same principle applies to ranges reciting minimal or maximal values. Furthermore, such an interpretation should apply regardless of the breadth of the range or the characteristics being described.

The term **"about"** when used in connection with a numerical value is meant to encompass numerical values within a range having a lower limit that is 5% smaller than the indicated numerical value and having an upper limit that is 5% larger than the indicated numerical value.

In the context of the present disclosure, the term **"analyte",** "analyte molecule" , or "analyte(s) of interest" are used interchangeably referring the chemical specis to be analysed via mass spectrometry. Chemical species suitable to be analysed via mass spectrometry, i.e. analytes, can be any kind of molecule present in a living organism, include but are not limited to nucleic acid (e.g. DNA, mRNA, miRNA, rRNA etc.), amino acids, peptides, proteins (e.g. cell surface receptor, cytosolic protein etc.), metabolite or hormones (e.g. testosterone, estrogen, estradiol, etc.), fatty acids, lipids, carbohydrates, steroids, ketosteroids, secosteroids (e.g. Vitamin D), molecules characteristic of a certain modification of another molecule (e.g. sugar moieties or phosphoryl residues on proteins, methyl-residues on genomic DNA) or a substance that has been internalized by the organism (e.g. therapeutic drugs, drugs of abuse, toxin, etc.) or a metabolite of such a substance. Such analyte may serve as a biomarker. In the context of present invention, the term "biomarker" refers to a substance within a biological system that is used as an indicator of a biological state of said system.

Analytes or an analyte of interest may be present in a biological or clinical sample. The term **"sample or biological sample or clinical sample"** are used interchangeably herein, referring to a part or piece of a tissue, organ or individual, typically being smaller than such tissue, organ or individual, intended to represent the whole of the tissue, organ or individual. Upon analysis a biological or clinical sample provides information about the tissue status or the health or diseased status of an organ or individual. Examples of biological or clinical samples include but are not limited to fluid samples such as blood, serum, plasma, synovial fluid, spinal fluid, urine, saliva, and lymphatic fluid, or solid biological or clinical samples such as dried blood spots and tissue extracts. Further examples of biological or clinical samples are cell cultures or tissue cultures.

The term **"determining an analyte or determining at least one analyte"** as used herein is a broad term and is to be given its ordinary and customary meaning to a person of ordinary skill in the art and is not to be limited to a special or customized meaning. The term specifically may refer, without limitation, to a quantitative and/or qualitative determination of at least one analyte in an arbitrary sample. The quantitative and/or qualitative determination of the analyte in the sample may be a result or an intermediate result of a detection process that may comprise at least one measurement step as well as further steps such as at least one preparation step and/or at least one analyzing step. As part of the detection process at least one measurement value may be generated, specifically a measurement value regarding the presence, absence, concentration or amount of the analyte in the sample.

The term **"providing",** as used herein, is a broad term and is to be given its ordinary and customary meaning to a person of ordinary skill in the art and is not to be limited to a special or customized meaning. The term specifically may refer, without limitation, to a process of making available one or more needed objects.

The term **"microparticle"** as used herein is a broad term and is to be given its ordinary and customary meaning to a person of ordinary skill in the art and is not to be limited to a special or customized meaning. The term specifically may refer, without limitation, to an arbitrary particulate matter of microscopic size. The microparticles may have a mean diameter in the range from 100 nm to 100 µm, specifically from 200 nm to 50 µm. The microparticles may also be referred to as beads. The microparticles may be of spherical or globular shape. However, slight derivations from the spherical or globular shape may be feasible. The size od the microparticle can be determined by dynamic light scattering.

As outlined above, the microparticles have the at least one microparticle surface. The term **"microparticle surface"** and/or the term **"substrate surface"** as used herein is a broad term and is to be given its ordinary and customary meaning to a person of ordinary skill in the art and is not to be limited to a special or customized meaning. The term specifically may refer, without limitation, to an entirety of areas which delimit an arbitrary body from the outside. Thus, the body, e.g. the microparticle and/or substrate, may have a plurality of surfaces. Specifically, the microparticles may have a core surrounded by the surface. The surface and the core may comprise different materials. Further, the surface and the core may have different properties. Exemplarily, the core may be magnetic. The surface may be configured for capturing molecules, e.g. a broad range of polar to apolar molecules, when the microparticles are incubated with a sample comprising such molecules.

The term **"incubation"** as used herein is a broad term and is to be given its ordinary and customary meaning to a person of ordinary skill in the art and is not to be limited to a special or customized meaning. The term specifically may refer, without limitation, to a mixing of at least two substances and/or to an addition of at least one substance to another. Specifically, a solid or particulate matter may be added to and/or mixed with a sample of liquid. Apart from the process of adding and/or mixing, the incubation may further comprise a period of time referred to as incubation time. During the incubation time one of the two substances may be adsorbed on a surface of the other one of the two substances. During the incubation time further conditions, such as temperature and/or other conditions, may be chosen e.g. to favor the desired adsorption. Thus, in step b) the microparticles may be added to the sample and may optionally be mixed with the sample. In step b), the sample may be incubated with the microparticles with an incubation time of 1 s to 60 min, preferably of 1 min to 30 min, most preferably of 3 min to 12 min. However, also other durations may be feasible.

The term **"being adsorbed on a surface"** as used herein is a broad term and is to be given its ordinary and customary meaning to a person of ordinary skill in the art and is not to be limited to a special or customized meaning. The term specifically may refer, without limitation, to a result of a process during which atoms, ions or molecules forming part of a gas or liquid accumulate at a surface of an object of solid or particulate matter. The atoms, ions or molecules that may initially be distributed throughout the gas or liquid may be attracted by the surface of the solid matter or the particulate matter in the process of adsorption.

The term **"analyte-microparticle-complex"** as used herein is a broad term and is to be given its ordinary and customary meaning to a person of ordinary skill in the art and is not to be limited to a special or customized meaning. The term specifically may refer, without limitation, to an ensemble comprising at least one microparticle and at least one analyte, specifically one microparticle and a plurality of analytes. The microparticle and the analyte, specifically the analytes, forming the complex may be reversibly associated. Thus, the components of the complex may, at least under certain conditions, leave the complex or dissociate from the complex. The analyte-microparticle-complex may form on the basis of at least one force of attraction between the microparticle and the analyte. In particular, the force of attraction may act between the surface of the microparticles and the analyte. Thus, the analyte that may initially be distributed in the sample, specifically in a liquid phase of the sample, may accumulate in a process of adsorption at the surface of the microparticles. The forces of attraction may include van der Waals forces and electrostatic attraction. Other forces of attraction are feasible. E.g. the forces of attraction may include covalently bounding, in particular if immunobeads and the analyte forms the analyte-microparticle complex. Specifically, at least one chemical bond may be formed between the microparticle and the analyte, specifically between the surface of the microparticle and the analyte, as part of the formation of the analyte-microparticle-complex. The analyte-microparticle-complex may also be referred to as analyte loaded microparticles.

The term **"contacting"** as used herein is a broad term and is to be given its ordinary and customary meaning to a person of ordinary skill in the art and is not to be limited to a special or customized meaning. The term specifically may refer, without limitation, to a directly or indirectly connection between the analyte-microparticle-complex and the ionization matrix in order to form a matrix:analyte-microparticle sample. Contacting may also be described by either co-crystallization and/or mixing with the ionization matrix.

The term "vice versa" as used herein is a broad term and is to be given its ordinary and customary meaning to a person of ordinary skill in the art and is not to be limited to a special or customized meaning. The term specifically may refer, without limitation, to the addition of the dissolved analyte-microparticlecomplex to the ionization matrix. Alternantively, the ionization matrix can be added to the dissolved analyte-microparticle complex.

The term "mechanical ionization" as used herein is a broad term and is to be given its ordinary and customary meaning to a person of ordinary skill in the art and is not to be limited to a special or customized meaning. The term specifically may refer, without limitation, to a process which generates ionization energy by a mechanical process. Additionally or alternatively, the term specifically may refer, without limitation, to a transfer of energy from the matrix to the analyte, that was previously generated by an induction of mechanical force to the matrix. This mechanical force can be caused by shear force and/or triboluminescence of the respective crystals.

The term "triboluminescent matrix" as used herein is a broad term and is to be given its ordinary and customary meaning to a person of ordinary skill in the art and is not to be limited to a special or customized meaning. The term specifically may refer, without limitation, to a matrix which generated a discharge or generated an energetic discharge or electric discharge when the matrix is mechanically pulled apart, ripped, scratched, crushed, or rubbed.

The term "heterogenic solid **liquid** phase" as used herein is a broad term and is to be given its ordinary and customary meaning to a person of ordinary skill in the art and is not to be limited to a special or customized meaning. The term specifically may refer, without limitation, to the presence of a crystalline phase or a semicrystalline phase.

The term "automatically" or "automated" as used herein is a broad term and is to be given its ordinary and customary meaning to a person of ordinary skill in the art and is not to be limited to a special or customized meaning. The term specifically may refer, without limitation, to a process which is performed completely by means of at least one computer and/or computer network and/or machine, in particular without manual action and/or interaction with a user.

The term **"fully automated"** may refer to a process which is performed completely by means of at least one computer and/or computer network and/or machine, without manual action and/or interaction with a user.

The term **"partially automated"** may refer to a process which is performed by means of at least one computer and/or computer network and/or machine and with the aid of manual action and/or interaction with a user. Preferably, "partially automated" can mean that the manual action and/or interaction with a user is 50% or 40% or 30% or 20% or 10% or 5% at the maximum of the total process, wherein the rest of the process is performed by means of at least one computer and/or computer network and/or machine. "By means of at least one computer and/or computer network and/or machine" can mean that this process is performed without any manual action and/or interaction with a user.

The term "Mass **Spectrometry"** ("Mass Spec" or "MS") or "mass spectrometric determination" or "mass spectrometric analysis" relates to an analytical technology used to identify compounds by their mass. MS is a methods of filtering, detecting, and measuring ions based on their mass-to-charge ratio, or "m/z". MS technology generally includes (1) ionizing the compounds to form charged compounds; and (2) detecting the molecular weight of the charged compounds and calculating a mass-to-charge ratio. The compounds may be ionized and detected by any suitable means. A "mass spectrometer" generally includes an ionizer and an ion detector. In general, one or more molecules of interest are ionized, and the ions are subsequently introduced into a mass spectrographic instrument where, due to a combination of magnetic and electric fields, the ions follow a path in space that is dependent upon mass ("m") and charge ("z"). The term "ionization" or "ionizing" refers to the process of generating an analyte ion having a net charge equal to one or more units. Negative ions are those having a net negative charge of one or more units, while positive ions are those having a net positive charge of one or more units. The MS method may be performed either in "negative ion mode", wherein negative ions are generated and detected, or in "positive ion mode" wherein positive ions are generated and detected.

**"Tandem mass spectrometry"** or "MS/MS" involves multiple steps of mass spectrometry selection, wherein fragmentation of the analyte occurrs in between the stages. In a tandem mass spectrometer, ions are formed in the ion source and separated by mass-to-charge ratio in the first stage of mass spectrometry (MS1). Ions of a particular mass-to-charge ratio (precursor ions or parent ion) are selected and fragment ions (or daughter ions) are created by collision-induced dissociation, ion-molecule reaction, or photodissociation. The resulting ions are then separated and detected in a second stage of mass spectrometry (MS2).

Since a mass spectrometer separates and detects ions of slightly different masses, it easily distinguishes different isotopes of a given element. Mass spectrometry is thus, an important method for the accurate mass determination and characterization of analytes, including but not limited to low-molecular weight analytes, peptides, polypeptides or proteins. Its applications include the identification of proteins and their post-translational modifications, the elucidation of protein complexes, their subunits and functional interactions, as well as the global measurement of proteins in proteomics. De novo sequencing of peptides or proteins by mass spectrometry can typically be performed without prior knowledge of the amino acid sequence.

Most sample workflows in MS further include sample preparation and/or enrichment steps, wherein e.g. the analyte(s) of interest are separated from the matrix using e.g. gas or liquid chromatography. Typically, for the mass spectrometric measurement, the following three steps are performed:
1. a sample comprising an analyte of interest is ionized, e.g. via matrix-assisted ionization (MAI).
2. the ions are sorted and separated according to their mass and charge. For example, high-field asymmetric-waveform ion-mobility spectrometry (FAIMS) may be used as ion filter.
3. the separated ions are then detected, e.g. in multiple reaction mode (MRM), and the results are displayed on a chart.

The term "matrix-assisted ionization or inlet ionization" can mean a low fragmentation (soft) ionization technique which involves the transfer of particles of the analyte and matrix sample from atmospheric pressure (AP) to the heated inlet tube connecting the AP region to the vacuum of the mass analyzer.

"High-field asymmetric-waveform ion-mobility spectrometry (FAIMS)" is an atmospheric pressure ion mobility technique that separates gas-phase ions by their behavior in strong and weak electric fields.

"Multiple reaction mode" or "MRM" is a detection mode for a MS instrument in which a precursor ion and one or more fragment ions arc selectively detected.

Mass spectrometric determination may be combined with additional analytical methods including chromatographic methods such as gas chromatography (GC), liquid chromatography (LC), particularly HPLC, and/or ion mobility-based separation techniques. In a preferred embodiment, the mass spectrometric determination is free of additional analytical methods including chromatographic methods such as gas chromatography (GC), liquid chromatography (LC), particularly HPLC, and/or ion mobility-based separation techniques.

Before being analysed via Mass Spectrometry, a sample may be pre-treated in a sample- and/or analyte specific manner. In the context of the present disclosure, the term "pre-treatment" refers to any measures required to allow for the subsequent analysis of a desired analyte via Mass Spectrometry. Pre-treatment measures typically include but are not limited to the elution of solid samples (e.g. elution of dried blood spots), addition of hemolizing reagent (HR) to whole blood samples, and the addition of enzymatic reagents to urine samples. Also the addition of internal standards (ISTD) is considered as pre-treatment of the sample.

The term "hemolysis reagent" (HR) refers to reagents which lyse cells present in a sample, in the context of this invention hemolysis reagents in particular refer to reagents which lyse the cell present in a blood sample including but not limited to the erythrocytes present in whole blood samples. A well known hemolysis reagent is water (H₂O). Further examples of hemolysis reagents include but are not limited to deionized water, liquids with high osmolarity (e.g. 8M urea), ionic liquids, and different detergents.

Typically, an "internal standard" (ISTD) is a known amount of a substance which exhibits similar properties as the analyte of interest when subjected to the mass spectrometric detection worklflow (i.e. including any pre-treatment, enrichment and actual detection step). Although the ISTD exhibits similar properties as the analyte of interest, it is still clearly distinguishable from the analyte of interest. Exemplified, during chromatographic separation, such as gas or liquid chromatography, the ISTD has about the same retention time as the analyte of interest from the sample. Thus, both the analyte and the ISTD enter the mass spectrometer at the same time. The ISTD however, exhibits a different molecular mass than the analyte of interest from the sample. This allows a mass spectrometric distinction between ions from the ISTD and ions from the analyte by means of their different mass/charge (m/z) ratios. Both are subject to fragmentation and provide daughter ions. These daughter ions can be distinguished by means of their m/z ratios from each other and from the respective parent ions. Consequently, a separate determination and quantification of the signals from the ISTD and the analyte can be performed. Since the ISTD has been added in known amounts, the signal intensity of the analyte from the sample can be attributed to a specific quantitative amount of the analyte. Thus, the addition of an ISTD allows for a relative comparison of the amount of analyte detected, and enables unambiguous identification and quantification of the analyte(s) of interest present in the sample when the analyte(s) reach the mass spectrometer. Typically, but not necessarily, the ISTD is an isotopically labeled variant (comprising e.g. ²H, ¹³C, or ¹⁵N etc. label) of the analyte of interest.

In addition to the pre-treatment, the sample may also be subjected to one or more enrichment steps. In the context of the present disclosure, the term "first enrichment process" or "first enrichment workflow" refers to an enrichment process which occurs subsequent to the pre-treatment of the sample and provides a sample comprising an enriched analyte relative to the initial sample. The first enrichment workflow may comprise chemical precipitation (e.g. using acetonitrile) or the use of a solid phase. Suitable solid phases include but are not limited to Solid Phase Extraction (SPE) cartridges, and beads. Beads may be non-magnetic, magnetic, or paramagnetic. Beads may be coated differently to be specific for the analyte of interest. The coating may differ depending on the use intended, i.e. on the intended capture molecule. It is well-known to the skilled person which coating is suitable for which analyte. The beads may be made of various different materials. The beads may have various sizes and comprise a surface with or without pores. The beads may be immunofunctionalized.

In the context of the present disclosure the term **"second enrichment process"** or "second enrichment workflow" refers to an enrichment process which occurs subsequent to the pre-treatment and the first enrichment process of the sample and provides a sample comprising an enriched analyte relative to the initial sample and the sample after the first enrichment process.

The term **"chromatography"** refers to a process in which a chemical mixture carried by a liquid or gas is separated into components as a result of differential distribution of the chemical entities as they flow around or over a stationary liquid or solid phase. In embodiments of the present invention, the method or sample element or device or kit are free of a chromatography step and chromatography unit, respectively.

The term **"liquid chromatography"** or "LC" refers to a process of selective retardation of one or more components of a fluid solution as the fluid uniformly percolates through a column of a finely divided substance, or through capillary passageways. The retardation results from the distribution of the components of the mixture between one or more stationary phases and the bulk fluid, (i.e., mobile phase), as this fluid moves relative to the stationary phase(s). Methods in which the stationary phase is more polar than the mobile phase (e.g., toluene as the mobile phase, silica as the stationary phase) are termed normal phase liquid chromatography (NPLC) and methods in which the stationary phase is less polar than the mobile phase (e.g., water-methanol mixture as the mobile phase and C18 (octadecylsilyl) as the stationary phase) is termed reversed phase liquid chromatography (RPLC).

**"High** performance **liquid** chromatography" or "HPLC" refers to a method of liquid chromatography in which the degree of separation is increased by forcing the mobile phase under pressure through a stationary phase, typically a densely packed column. Typically, the column is packed with a stationary phase composed of irregularly or spherically shaped particles, a porous monolithic layer, or a porous membrane. HPLC is historically divided into two different sub-classes based on the polarity of the mobile and stationary phases. Methods in which the stationary phase is more polar than the mobile phase (e.g., toluene as the mobile phase, silica as the stationary phase) are termed normal phase liquid chromatography (NPLC) and the opposite (e.g., water-methanol mixture as the mobile phase and C18 (octadecylsilyl) as the stationary phase) is termed reversed phase liquid chromatography (RPLC). Micro LC refers to a HPLC method using a column having a norrow inner column diameter, typically below 1 mm, e.g. about 0.5 mm. "Ultra high performance liquid chromatography" or "UHPLC" refers to a HPLC method using a pressure of 120 MPa (17,405 lbf/in2), or about 1200 atmospheres. Rapid LC refers to an LC method using a column having an inner diameter as mentioned above, with a short length <2 cm, e.g. 1 cm, applying a flow rate as mentioned above and with a pressure as mentioned above (Micro LC, UHPLC). The short Rapid LC protocol includes a trapping / wash / elution step using a single analytical column and realizes LC in a very short time <1 min.

Further well-known LC modi include hydrophilic interaction chromatography (HILIC), size-exclusion LC, ion exchange LC, and affinity LC.

LC separation may be single-channel LC or multi-channel LC comprising a plurality of LC channels arranged in parallel. In LC analytes may be separated according to their polarity or log P value, size or affinity, as generally known to the skilled person.

The term "ion mobility spectrometry" as used herein is a broad term and is to be given its ordinary and customary meaning to a person of ordinary skill in the art and is not to be limited to a special or customized meaning. The term specifically may refer, without limitation, to a device for a separation of ions in an electric field and in the presence of at least one buffer gas, based on the mobility characteristics of the analyte ions.

The term "crystallized" as used herein is a broad term and is to be given its ordinary and customary meaning to a person of ordinary skill in the art and is not to be limited to a special or customized meaning. The term specifically may refer, without limitation, to a formation of higly organized solid structures of molecules out of a supersaturated liquid solution, that can additionally include different analyte molecules into their structures.

The term "pre-crystallized" as used herein is a broad term and is to be given its ordinary and customary meaning to a person of ordinary skill in the art and is not to be limited to a special or customized meaning. The term specifically may refer, without limitation, to the crystallization process of the ionization matrix prior to the addition to the analyte-bead mixture.

In embodiments, the term "crystallized" and "pre-crystallized" can be used interchangeable.

A "clinical diagnostics system" is a laboratory automated apparatus dedicated to the analysis of samples for in vitro diagnostics. The clinical diagnostics system may have different configurations according to the need and/or according to the desired laboratory workflow. Additional configurations may be obtained by coupling a plurality of apparatuses and/or modules together. A "module" is a work cell, typically smaller in size than the entire clinical diagnostics system, which has a dedicated function. This function can be analytical but can be also pre-analytical or post analytical or it can be an auxiliary function to any of the pre-analytical function, analytical function or post-analytical function. In particular, a module can be configured to cooperate with one or more other modules for carrying out dedicated tasks of a sample processing workflow, e.g. by performing one or more pre-analytical and/or analytical and/or post-analytical steps. In particular, the clinical diagnostics system can comprise one or more analytical apparatuses, designed to execute respective workflows that are optimized for certain types of analysis, e.g. clinical chemistry, immunochemistry, coagulation, hematology, liquid chromatography separation, mass spectrometry, etc. Thus the clinical diagnostic system may comprise one analytical apparatus or a combination of any of such analytical apparatuses with respective workflows, where pre-analytical and/or post analytical modules may be coupled to individual analytical apparatuses or be shared by a plurality of analytical apparatuses. In alternative pre-analytical and/or post-analytical functions may be performed by units integrated in an analytical apparatus. The clinical diagnostics system can comprise functional units such as liquid handling units for pipetting and/or pumping and/or mixing of samples and/or reagents and/or system fluids, and also functional units for sorting, storing, transporting, identifying, separating, detecting. The clinical diagnostic system can comprise a sample preparation station for the automated preparation of samples comprising analytes of interest, optionally a liquid chromatography (LC) separation station comprising a plurality of LC channels and/or optionally a sample preparation/LC interface for inputting prepared samples into any one of the LC channels. The clinical diagnostic system can further comprise a controller programmed to assign samples to pre-defined sample preparation workflows each comprising a pre-defined sequence of sample preparation steps and requiring a pre-defined time for completion depending on the analytes of interest. The clinical diagnostic system can further comprise a mass spectrometer (MS) and an LC/MS interface for connecting the LC separation station to the mass spectrometer.

A "sample **preparation** station" can be a pre-analytical module coupled to one or more analytical apparatuses or a unit in an analytical apparatus designed to execute a series of sample processing steps aimed at removing or at least reducing interfering matrix components in a sample and/or enriching analytes of interest in a sample. Such processing steps may include any one or more of the following processing operations carried out on a sample or a plurality of samples, sequentially, in parallel or in a staggered manner: pipetting (aspirating and/or dispensing) fluids, pumping fluids, mixing with reagents, incubating at a certain temperature, heating or cooling, centrifuging, separating, filtering, sieving, drying, washing, resuspending, aliquoting, transferring, storing, etc.).

The clinical diagnostic system, e.g. the sample preparation station, may also comprise a buffer unit for receiving a plurality of samples before a new sample preparation start sequence is initiated, where the samples may be individually randomly accessible and the individual preparation of which may be initiated according to the sample preparation start sequence.

The clinical diagnostic system makes use of mass spectrometry more convenient and more reliable and therefore suitable for clinical diagnostics. In particular, high-throughput, e.g. up to 100 samples/hour or more with random access sample preparation and LC separation can be obtained while enabling online coupling to mass spectrometry. Moreover the process can be fully automated increasing the walk-away time and decreasing the level of skills required.

A "kit" is any manufacture (e.g., a package or container) comprising at least one reagent, e.g., a medicament for treatment of a disorder, or a probe for specifically detecting a biomarker gene or protein of the invention. The kit is preferably promoted, distributed, or sold as a unit for performing the method of the present invention. Typically, a kit may further comprise carrier means being compartmentalized to receive in close confinement one or more container means such as vials, tubes, and the like. In particular, each of the container means comprises one of the separate elements to be used in the method of the first aspect. Kits may further comprise one or more other reagents including but not limited to reaction catalyst. Kits may further comprise one or more other containers comprising further materials including but not limited to buffers, internal standard, diluents, filters, needles, syringes, and package inserts with instructions for use. A label may be present on the container to indicate that the composition is used for a specific application, and may also indicate directions for either in vivo or in vitro use. The computer program code may be provided on a data storage medium or device such as a optical storage medium (e.g., a Compact Disc) or directly on a computer or data processing device. Moreover, the kit may, comprise standard amounts for the biomarkers as described elsewhere herein for calibration purposes.

### Embodiments

In a first aspect, the present invention relates to a method for determining at least one analyte in a sample according to claim 1.

The inventors surprisingly found that subject matters of the present invention, in particular the method according to the first aspect of the invention, show a simple and robust way to overcome the above-mentioned disadvantages.

The principles of matrix ionization also works if a double solid supported workflow is applied. Surprisingly the inventors found that even if the analyte is adsorbed on a solid support, in particular a microparticle, and the ionization matrix is on a solid, preferably a substrate, e.g. paper tissue, together with the microparticles (e.g. in paper sucked in analyte loaded microparticles and crystalline ionization matrix component simultaneously) the ionization process is still working for a huge variety of analytes.

This method has the advantage that no elution step to desorb the analyte from the microparticle is necessary. The analyte loaded microparticles can be washed and the analyte loaded microparticle together with a (pre-crystallized) ionization matrix sucked in a paper issue. The microparticles on the paper tissue can be dried and stored and late be analyzed. This a can be called a dried bead spot.

**In** a first aspect of the present invention, a method for determining at least one analyte in a sample is disclosed.

According to step a), the at least one analyte, at least one microparticle, at least one ionization matrix, and a substrate having a substrate surface are provided.

According to step b), the analyte with the microparticle having at least one microparticle surface is incubated. The analyte is adsorbed on the surface of the microparticle and an analyte-microparticle-complex is formed. **In** this context, the expression can be understood that a plurality of analyte-microparticle-complexes are formed. This, in step b), the sample may be incubated with the microparticles having the at least one surface whereby the analyte is adsorbed on the surface of the microparticles and the analyte-microparticle-complexes are formed.

**In** embodiments of the first aspect of the invention, the microparticle can be modified by chemicals, which are selected from the group consisting of hydrophobic compounds, hydrophilic compounds, immune chemistry compounds.

In embodiments of the first aspect, the hydrophobic compounds are e.g. compounds having carboxylic and/or alkyl groups.

In embodiments of the first aspect, the hydrophilic compounds are compounds having e.g hydroxylic functions.

In embodiments of the first aspect, the immune chemistry compounds are e.g. compounds having specific antibodies.

In embodiments of the first aspect, the microparticle is a magnetic particle.

In embodiments of the first aspect, the microparticle is a magnetic particle, which is coated, wherein the coating is a glass coating or a polymer coating.

In embodiments of the first aspect, the microparticle is an immunobead for immobilization of antibodies.

In embodiments of the first aspect, the microparticle is a protein coated, e.g. streptavidin coated, magnetic bead.

In embodiments of the first aspect, the microparticle is selected from the group consisting of: magnetic microparticle; silica microparticle; melamine resin microparticle; poly(styrene) based microparticle; poly(methyl methacrylate) microparticle.

In particular, a microparticle or microparticles may be selected from the group consisting of: magnetic microparticles, specifically magnetic microparticles having a magnetic core and a modified surface; silica microparticles, specifically silica microparticles having a silica core and a modified surface; melamine resin microparticles, specifically melamine resin microparticles having a melamine resin core and a modified surface; poly(styrene) based microparticles, specifically poly(styrene) based microparticles having a poly(styrene) core and a modified surface; poly(methyl methacrylate) microparticles, specifically poly(methyl methacrylate) microparticles having a poly(methyl methacrylate) core and a modified surface. However, also other particles may be feasible. The melamine resin microparticles may have a mean diameter of 500 nm to 20 µm, preferably of 2 µm to 4 µm, most preferably of 3 µm. The poly(styrene) based microparticles may have a mean diameter of 500 nm to 50 µm, preferably of 2 µm to 4 µm, most preferably of 3 µm. The poly(methyl methacrylate) microparticles may have a mean diameter of 500 nm to 50 µm, preferably of 2 µm to 4 µm, most preferably of 3 µm. The modified surface of the magnetic microparticles may be a modified poly(styrene) surface and the magnetic microparticles may have a mean diameter of 5 µm to 50 µm, preferably of 10 µm to 30 µm, most preferably of 20 µm. The modified surface of the magnetic microparticles may be a silica surface and the magnetic microparticles may have a mean diameter of 100 nm to 1000 nm, preferably of 200 nm to 500 nm, most preferably of 300 nm. The modified surface of the silica microparticles may be a cyanopropyl silane functionalized surface and the silica microparticles may have a mean diameter of 5 µm to 100 µm, preferably of 20 µm to 80 µm, most preferably of 40 µm. Also other dimensions may be feasible.

In embodiments of the first aspect, the microparticle is a magnetic particle.

In embodiments of the first aspect, the microparticle is a magnetic particle comprising a polymer surface and at least one magnetic core, wherein the polymer surface comprises a hypercrosslinked polymer and wherein the magnetic particle has a particle size in the range of from 5 to 40 micrometers, as determined according to ISO 13320. For the term "polymer surface" can aslo be used "polymer matrix".

In embodiments of the first aspect, the polymer surface comprises pores having a pore size smaller than 100 nm, preferably smaller or equal to 50 nm, as determined according to ISO 15901-3.

In embodiments of the first aspect, the particle has a BET specific surface area in the range of from 50 to 2500 m /g, as determined according to ISO 9277.

In embodiments of the first aspect, the magnetic particle has a saturation magnetization of at least 1 A m /kg, preferably, of at least 10 A m /kg.

In embodiments of the first aspect, the at least one magnetic core comprises at least one magnetic nanoparticle, preferably at least one iron oxide nanoparticle, more preferably a Fe₃O₄-nanoparticle.

In embodiments of the first aspect, the magnetic core comprises, more preferably consists of, at least one nanoparticle and a coating C1.

In embodiments of the first aspect, the at least one magnetic core comprises, preferably consists of, a supraparticle and, optionally, comprising a coating C1.

In embodiments of the first aspect, the at least one coating C1 is selected from the group consisting of tensides, silica, silicates, silanes, phosphates, phosphonates, phosphonic acids and mixtures of two or more thereof.

In embodiments of the first aspect, the polymer surface comprises a co-polymer obtained or obtainable by a method comprising co-polymerizing suitable monomeric building blocks in the presence of at least one monomeric building block which is a crosslinking agent, wherein preferably 5-90 vol% of all monomeric building blocks are crosslinking agents, more preferably divinylbenzenes.

In embodiments of the first aspect, the microparticle is supramagnetic.

In embodiments of the first aspect, the hypercrosslinked polymer can be produced by hypercrosslinking, wherein the hypercrosslinking is carried out in the presence of a catalyst selected from the group consisting of a Lewis acid, preferably selected from the group consisting of FeCl₃, ZnCl₂, AlCl₃, BF₃, SbCl₅, SnCl₄, TiCl₄, SiCl₄ and mixtures of two or more thereof, more preferably FeCl₃ or ZnCl₂, or a mixture thereof.

In embodiments of the first aspect, the method comprises the following steps:
b1) separating of the analyte-microparticle-complex, specifically of the analyte-microparticle-complexes, from further components of the sample; and
b2) removing the further components of the sample from the analyte-microparticle complex, specifically from the analyte-microparticle complexes.

In embodiments of the first aspect, the method comprises the following step:
b3) washing the analyte-microparticle-complex, specifically the analyte-microparticle-complexes.

Specifically, the analyte-microparticle-complex may be washed with a solvent or washing solvent. A composition of the washing solvent may be chosen such that the analyte remains bound to the microparticle. The washing solvent may be or may comprise deionized water. Further, the washing solvent may comprise a mixture of water, one or more buffering salts, one or more pH-adjusting additives and/or one or more organic solvents. The organic solvent may be selected from the group consisting of: methanol, ethanol, isopropanol, acetonitrile. A content of the organic solvent may be 0 vol% to 10 vol%. The step b3) may be repeated at least two times, preferably at least three times.

According to step c), the analyte-microparticle-complex with the ionization matrix are contacted to form a matrix:analyte-microparticle sample.

In embodiments of the first aspect of the invention, step c) comprises:
c1) Providing the analyte-microparticle-complex dissolved in a solvent, then
c2) Adding the ionization matrix to the dissolved analyte-microparticle-complex or vice versa to form a matrix:analyte-microparticle sample, and then
c3) Applying the matrix:analyte-microparticle sample on the substrate surface, wherein the ionization matrix in step c2) is crystallized or dissolved in a further solvent, wherein the solvent and the further solvent can be the same or different.

In embodiments of the first aspect of the invention, step c) comprises:
c4) Providing the analyte-microparticle-complex dissolved in a solvent, then
c5) Applying the dissolved analyte-microparticle-complex on a substrate surface, and then
c6) Adding the ionization matrix to the dissolved analyte-microparticle-complex to form a matrix:analyte-microparticle sample, wherein the ionization matrix in step c6) is crystallized or dissolved in a further solvent, wherein the solvent and the further solvent can be the same or different.

In embodiments of the first aspect of the invention, the ionization matrix is crystallized at least in step c).

In embodiments of the first aspect of the invention, the analyte-microparticle-complex is in a fluid state in step c) and/or in a solid state by performing step f).

According to step d), matrix:analyte-microparticle sample is provided on the substrate surface.

According to step e), at least the analyte is ionized. The ionization is a mechanical ionization.

The ionization in step e) is induced by a mechanical force, which comprises or consists of a shear force and/or wherein the mechanical ionization is induced by mechanical stimulation, wherein the mechanical stimulation is triboluminescence.

The ionization in step e) is mechanical ionization, wherein the mechanical ionization is induced by shear force and/or triboluminescence.

In embodiments of the first aspect of the invention, the mechanical ionization is induced by a mechanical force, which preferably comprises or consists of a shear force. Preferably, the mechanical force is caused by shear force and/or triboluminescence of the respective crystals.

In embodiments of the first aspect of the invention, the mechanical ionization is induced by mechanical stimulation, preferably wherein the mechanical stimulation is triboluminescence.

In embodiments of the first aspect of the invention, the mechanical ionization is induced by a mechanical force, which preferably comprises or consists of a shear force and/or the mechanical ionization is induced by mechanical stimulation, preferably wherein the mechanical stimulation is triboluminescence.

In embodiments of the first aspect of the invention, the ionization in step e) is a matrix assisted ionization (MAI), preferably a double solid supported matrix assisted ionization. For example, the analyte is adsorbed on a solid support, in particular a microparticle, and the ionization matrix is on a solid, preferably a substrate, e.g. paper tissue, together with the microparticles (e.g. in paper sucked in analyte loaded microparticles and crystalline ionization matrix component simultaneously).

In embodiments of the first aspect of the invention, step e) is not induced by a laser.

In embodiments of the first aspect of the invention, the ionization matrix is a triboluminescent matrix.

In embodiments of the first aspect of the invention, the ionization matrix is selected from the group consisting of salsalate, 3-nitrobenzonitrile, 2,2'-azobis(2-methylpropane), 2-nitrobenzonitrile, 5-methyl-2- nitrobenzonitrile, coumarin, methyl-2-methyl-3-nitrobenzoate, methyl-5-nitro-2-furoate, 2-bromo-2-nitropropane-1,3-diol, 3-nitrobenzaldehyde, 6-nitro-o-anisonitrile, phthalic anhydride, or mixtures thereof.

In embodiments of the first aspect of the invention, the ionization matrix is in a heterogenic solid liquid phase at room temperature and pressure.

In embodiments of the first aspect of the invention, the ionization matrix undergoes a phase transfer under a sub-atmospheric pressure, preferably from solid phase to gaseous phase.

In embodiments of the first aspect of the invention, the ionization matrix undergoes a phase transfer, preferably from solid phase to gaseous phase when placed under a sub-atmospheric pressure at a temperature less than 120° C.

In embodiments of the first aspect of the invention, the ionization matrix undergoes a phase transfer, preferably from solid phase to gaseous phase when placed under a sub-atmospheric pressure at a temperature less than 70° C.

In embodiments of the first aspect of the invention, step d) is performed by placing the matrix:analyte-microparticle sample as a spot on the substrate surface.

In embodiments of the first aspect of the invention, the analyte comprises biological tissue, biological material, eatable goods, polymers, paintings, archaeological artifacts, artificial bone, skin, urine, or blood.

In embodiments of the first aspect of the invention, the sample comprises formic acid (FA).

In embodiments of the first aspect of the invention, the analyte of interest is selected from the group consisting of nucleic acid, amino acid, peptide, protein, metabolite, hormones, fatty acid, lipid, carbohydrate, steroid, ketosteroid, secosteroid, a molecule characteristic of a certain modification of another molecule, a substance that has been internalized by the organism, a metabolite of such a substance and combination thereof.

In embodiments of the first aspect of the invention, the solvent and/or the further solvent is water, methanol, ethanol, isopropanol, acetonitrile, tetrahydrofuran, chloroform, dimethylformamide, dimethyl sulfoxide, acetone, or mixtures thereof.

In embodiments of the first aspect of the invention, the analyte and the microparticle are bounded covalently to form the analyte-microparticle-complex.

In embodiments of the first aspect of the invention, the analyte is Vitamin D and the microparticle is an immunobead.

In embodiments of the first aspect of the invention, the mass spectrometer or ion mobility spectrometer comprises an inlet and a region near said inlet, wherein said region near said inlet is maintained at a sub-atmospheric pressure.

In embodiments of the first aspect of the invention, the inlet is a system through which the at least one analyte and/or the matrix:analyte-microparticle sample are injected or inserted into a chamber at vacuum and optionally heated to achieve vaporization.

In embodiments of the first aspect of the invention, the inlet comprises a truncated sample entrance and a filter.

In embodiments of the first aspect of the invention, the filter is a nylon mesh, a membrane, a metal grid. In principal, other polymer materials for the filter can be used, e.g. polyester mesh, poly(tetrafluoroethylene) filter membrane, polypropylene filter membrane, poly(ether ether ketone) filter membrane.

In embodiments of the first aspect of the invention, the filter is part of the truncated sample entrance.

In embodiments of the first aspect of the invention, the filter is exchangeable.

In embodiments of the first aspect of the invention, the filter is coated with the ionization matrix and/or matrix:analyte-microparticle sample and/or analyte-microparticle complex.

In embodiments of the first aspect of the invention, the sample is a biological sample, wherein the biological sample is selected from the group consisting of: blood, serum, plasma, saliva, ocular lens fluid, cerebral spinal fluid, sweat, urine, milk, ascites fluid, mucous, synovial fluid, peritoneal fluid, amniotic fluid, tissue, cells.

In embodiments of the first aspect of the invention, the substrate is selected from the group consisting of metal, paper, cloth, ribbon, glass, plastic, polymer, sodium dodecyl sulfate gel, agarose gel, paper chromatography plate, silica plate and woven fiber.

In embodiments of the first aspect of the invention, the substrate is a plate, e.g. a glass plate, or a filter, e.g. a triangle-shaped filter.

In embodiments of the first aspect of the invention, the method is automated, preferably fully or partially automated.

According to step f), the analyte is determined via ion mobility spectrometry and/or mass spectrometry.

In embodiments of the first aspect of the invention, the method further comprises at least one of the following steps:
g) Providing a filter that is arranged between the analyte-microparticle-complex according to step d) and the ion mobility spectrometry or mass spectrometry for preventing the microparticle from entering the ion mobility spectrometry or mass spectrometry, and/or
h) Washing the analyte-microparticle-complex at least after step b), preferably by using water as a washing reagent.

In embodiments of the first aspect of the invention, the filter has a mesh size, which is smaller than the particle size of the microparticle.

In a second aspect, the present invention relates to a system as defined by independent claim 5.

A first example, which is not claimed relates to the use of the method of the first aspect of the present invention for determining the at least one analyte of interest.

A second example, which is not claimed relates to a sample element for determining at least one analyte and suitable to perform a method according to the first aspect of the present invention comprising
- a substrate surface,
- an ionization matrix arranged on the substrate surface and for using matrix-assisted ionization,
- an analyte-microparticle-complex arranged on the substrate surface,
- wherein the ionization matrix is selected from the group consisting of salsalate, 3-nitrobenzonitrile, 2,2'-azobis(2-methylpropane), 2-nitrobenzonitrile, 5-methyl-2- nitrobenzonitrile, coumarin, methyl-2-methyl-3-nitrobenzoate, methyl-5-nitro-2-furoate, 2-bromo-2-nitropropane-1,3-diol, 3-nitrobenzaldehyde, 6-nitro-o-anisonitrile, phthalic anhydride, or mixtures thereof,
- wherein the ionization matrix and/or analyte-microparticle-complex are crystallized or pre-crystallized,
- wherein the microparticle of the analyte-microparticle-complex is magnetic,
- wherein the analyte-microparticle-complex and ionization matrix are in contact to each other. All embodiments mentioned for the first aspect of the invention and/or second aspect of the invention apply for the third aspect of the invention and vice versa.

In embodiments of the second example, the ionization matrix is not induced by a laser or a laser ionization technique, e.g. MALDI or SALDI.

A third example, which is not claimed relates to an inlet for ion transport into the mass spectrometer or ion mobility spectrometer comprising a truncated sample entrance and a filter.

A fourth example, which is not claimed relates to the use of the inlet of the third example for determining the at least one analyte of interest.

In embodiments of the fourth example, the inlet is part of a device, preferably where the device is a clinical diagnostic system. This can mean that the device comprises the inlet.

In embodiments of the fourth example, the clinical diagnostic system comprises a sample preparation station.

A fifth example, which is not claimed relates to the use of the inlet of the fourth example for determining at least one analyte of interest.

A sixth example, which is not claimed relates to a composition for vacuum or inlet ionization comprising an ionization matrix, wherein the ionization matrix comprises or consists of salsalate.

In embodiments of the sixth example, the composition is for matrix assisted ionization (MAI), preferably for a double solid supported matrix assisted ionization.

In embodiments of the sixth example, the salsalate has the CAS number 552-94-3.

In embodiments of the sixth example, the salsalate has the following formula:

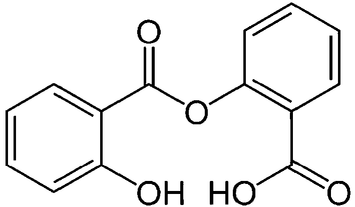

In embodiments of the sixth example, the compostion further comprises at least one analyte.

In embodiments of the sixth example, the compostion further comprises a microparticle, preferable a magnetic particle, which is coated, wherein the coating is a glass coating or a polymer coating.

In embodiments of the sixth example, the compostion further comprises a microparticle, preferable an immunobead.

In embodiments of the sixth example, the molar ratio of the ionization matrix and analyte is from 5:1 to 1x10⁷:1.

In embodiments of the sixth example, the composition comprises a matrix:analyte-microparticle sample or a matrix:analyte sample, wherein the ionization matrix:analyte sample or the matrix:analyte-microparticle sample is in a solid phase when exposed to sub-atmospheric pressure.

In embodiments of the sixth example, the ionization matrix is crystallized when performing vacuum or inlet ionization.

In embodiments of the sixth example, the ionization matrix:analyte-microparticle sample or the matrix:analyte sample is placed on a substrate, preferably as a spot.

In embodiments of the sixth example, the substrate is selected form the group consisting of metal, paper, cloth, ribbon, glass, plastic, polymer, sodium dodecyl sulfate gel, agarose gel, paper chromatography plate, silica plate or woven fiber.

In embodiments of the sixth example, the composition comprises a solvent.

In embodiments of the sixth example, the solvent is water, methanol, ethanol, isopropanol, acetonitrile, tetrahydrofuran, chloroform, dimethylformamide, dimethyl sulfoxide, acetone, or mixtures thereof.

In embodiments of the sixth example, the matrix:analyte-microparticle sample or the matrix:analyte sample is prepared by mixing or grinding the analyte and ionization matrix and optional the microparticle together.

In embodiments of the sixth example, the matrix:analyte-microparticle sample or matrix:analyte sample is a solid.

In embodiments of the sixth example, the solid sample is in a frozen state.

In embodiments of the sixth example, the matrix:analyte-microparticle sample or matrix:analyte sample further comprises an ammonium salt, metal salt, acid, base, or buffer.

A seventh example relates to the use of the composition of the sixth example, preferably in a method of the first aspect of the invention.

An eigth example relates to a kit suitable to perform a method according to the first aspect of the present invention comprising
(A) an ionization matrix,
(B) a solvent or a further solvent,
(C) a microparticle, and
(D) optionally at least one internal standard.

A ninth example relates to the use of a kit of the eigth example in a method according to the first aspect of the present invention.

In the following, further features are disclosed but not claimed:
The mechanical ionization may not be induced by evaporation or sublimation.

The method according may be automated.

Step e) may not be induced by a laser.

The ionization matrix may be crystallized at least in step c).

The ionization matrix may be a triboluminescent matrix.

Step c) may comprise:
c1) Providing the analyte-microparticle complex dissolved in a solvent, then
c2) Adding the ionization matrix to the dissolved analyte-microparticle-complex or vice versa to form a matrix:analyte-microparticle sample, and then
c3) Applying the matrix:analyte-microparticle sample on the substrate surface, wherein the ionization matrix in step c2) is crystallized or dissolved in a further solvent, wherein the solvent and the further solvent can be the same or different.

Step c) may comprise:
c4) Providing the analyte-microparticle-complex dissolved in a solvent, then
c5) Applying the dissolved analyte-microparticle-complex on a substrate surface, and then
c6) Adding the ionization matrix to the dissolved analyte-microparticle-complex to form a matrix:analyte-microparticle sample, wherein the ionization matrix in step c6) is crystallized or dissolved in a further solvent, wherein the solvent and the further solvent can be the same or different.

The method may further comprise at least one of the following steps:
g) Providing a filter that is arranged between the analyte-microparticle-complex according to step d) and the ion mobility spectrometry or mass spectrometry for preventing the microparticle from entering the ion mobility spectrometry or mass spectrometry, and/or
h) Washing the analyte-microparticle-complex at least after step b), preferably by using water as a washing reagent.

The filter may have a mesh size, which is smaller than the particle size of the microparticle.

The microparticle can be modified by chemicals, which are selected from the group consisting of hydrophobic compounds, hydrophilic compounds, immune chemistry compounds.

The microparticle may be an immunobead for immobilization of antibodies.

The microparticle may be a protein coated, e.g. streptavidin coated, magnetic bead.

The analyte-microparticle-complex may be in a fluid state in step c) and/or in a solid state by performing step f).

The microparticle may be selected from the group consisting of: magnetic microparticle; silica microparticle; melamine resin microparticle; poly(styrene) based microparticle; poly(methyl methacrylate) microparticle.

The microparticle may be a magnetic particle.

The microparticle may be a magnetic particle comprising a polymer surface (P) and at least one magnetic core (M), wherein the polymer surface comprises a hypercrosslinked polymer and wherein the magnetic particle has a particle size in the range of from 5 to 40 micrometers, as determined according to ISO 13320.

The polymer surface may comprise pores having a pore size smaller than 100 nm, preferably smaller or equal to 50 nm, as determined according to ISO 15901-3.

The particle may have a BET specific surface area in the range of from 50 to 2500 m /g, as determined according to ISO 9277.

The magnetic particle may have a saturation magnetization of at least 1A m /kg, preferably, of at least 10 A m /kg.

The at least one magnetic core (M) may comprise at least one magnetic nanoparticle, preferably at least one iron oxide nanoparticle, more preferably a Fe30 4-nanoparticle.

The magnetic core (M) may comprise, more preferably consists of, at least one nanoparticle and a coating C1.

The at least one magnetic core (M) may comprise, preferably consists of, a supraparticle and, optionally, comprising a coating C1.

The at least one coating C1 may be selected from the group consisting of tensides, silica, silicates, silanes, phosphates, phosphonates, phosphonic acids and mixtures of two or more thereof.

The polymer surface (P) may comprise a co-polymer obtained or obtainable by a method comprising co-polymerizing suitable monomeric building blocks in the presence of at least one monomeric building block which is a crosslinking agent, wherein preferably 5-90 vol% of all monomeric building blocks are crosslinking agents, more preferably divinylbenzenes.

The microparticle may be supramagnetic.

The hypercrosslinked polymer can be produced by hypercrosslinking, wherein the hypercrosslinking may be carried out in the presence of a catalyst selected from the group consisting of a Lewis acid, preferably selected from the group consisting of FeCl₃, ZnCl₂, AlCl₃, BF₃, SbCl₅, SnCl₄, TiCl₄, SiCl₄ and mixtures of two or more thereof, more preferably FeCl₃ or ZnCl₂, or a mixture thereof.

The sample may be a biological sample, wherein the biological sample may be selected from the group consisting of: blood, serum, plasma, saliva, ocular lens fluid, cerebral spinal fluid, sweat, urine, milk, ascites fluid, mucous, synovial fluid, peritoneal fluid, amniotic fluid, tissue, cells.

The substrate may be selected from the group consisting of metal, paper, cloth, ribbon, glass, plastic, polymer, sodium dodecyl sulfate gel, agarose gel, paper chromatography plate, silica plate and woven fiber.

The substrate may be a plate, e.g. a glass plate, or a filter, e.g. a triangle-shaped filter.

The ionization matrix may be in a heterogenic solid liquid phase at room temperature and pressure.

The ionization matrix may undergo a phase transfer under a sub-atmospheric pressure, preferably from solid phase to gaseous phase.

The ionization matrix may undergo a phase transfer, preferably from solid phase to gaseous phase when placed under a sub-atmospheric pressure at a temperature less than 120° C.

The ionization matrix may undergo a phase transfer, preferably from solid phase to gaseous phase when placed under a sub-atmospheric pressure at a temperature less than 70° C.

The analyte may comprise biological tissue, biological material, eatable goods, polymers, paintings, archaeological artifacts, artificial bone, skin, urine, or blood.

The sample may comprise formic acid (FA).

The method according to any of the proceeding aspects, wherein in step d) may be performed by placing the matrix:analyte-microparticle sample as a spot on the substrate surface.

The analyte of interest may be selected from the group consisting of nucleic acid, amino acid, peptide, protein, metabolite, hormones, fatty acid, lipid, carbohydrate, steroid, ketosteroid, secosteroid, a molecule characteristic of a certain modification of another molecule, a substance that has been internalized by the organism, a metabolite of such a substance and combination thereof.

The solvent and/or the further solvent may be water, methanol, ethanol, isopropanol, acetonitrile, tetrahydrofuran, chloroform, dimethylformamide, dimethyl sulfoxide, acetone, or mixtures thereof.

The analyte and the microparticle may be bounded covalently to form the analyte-microparticle-complex.

The analyte may be Vitamin D and the microparticle may be an immunobead or wherein the anaylte may be Testosterone and the microparticle may be an bead, e.g. immunobead.

The mass spectrometer or ion mobility spectrometer may comprise an inlet and a region near said inlet, wherein said region near said inlet may be maintained at a sub-atmospheric pressure.

The inlet may be a system through which the at least one analyte and/or the matrix:analyte-microparticle sample are injected or inserted into a chamber at vacuum and optionally heated to achieve vaporization.

The inlet may comprise a truncated sample entrance and a filter.

The filter may be a nylon mesh, a membrane, a metal grid.

The filter may be part of the truncated sample entrance.

The filter may be exchangeable.

The filter may be coated with the ionization matrix and/or matrix:analyte-microparticle sample and/or analyte-microparticle complex.

Use of the method according to an embodiement described herein for determining the at least one analyte of interest.

A sample element is disclosed for determining the at least one analyte and suitable to perform the method according to an embodiement described herein comprising
- a substrate surface,
- an ionization matrix arranged on the substrate surface and for using matrix-assisted ionization,
- an analyte-microparticle-complex arranged on the substrate surface,
- wherein the ionization matrix is selected from the group consisting of salsalate, 3-nitrobenzonitrile, 2,2'-azobis(2-methylpropane), 2-nitrobenzonitrile, 5-methyl-2- nitrobenzonitrile, coumarin, methyl-2-methyl-3-nitrobenzoate, methyl-5-nitro-2-furoate, 2-bromo-2-nitropropane-1,3-diol, 3-nitrobenzaldehyde, 6-nitro-o-anisonitrile, phthalic anhydride, or mixtures thereof,
- wherein the ionization matrix and/or analyte-microparticle-complex are crystallized,
- wherein the microparticle of the analyte-microparticle-complex is magnetic,
- wherein the analyte-microparticle-complex and ionization matrix are in contact to each other.

The ionization may not be induced by a laser.

A Use of the sample element according to an embodiement described herein is diosclosed for determining at least one analyte of interest, preferably in the method according to an embodiement described herein.

An inlet suitable is disclosed to perform the method according to an embodiement described herein and for ion transport into the mass spectrometer or ion mobility spectrometer or into the detector of the mass spectrometer or ion mobility spectrometer comprising a truncated sample entrance and a filter.

A Use of the inlet is disclosed for determining the at least one analyte of interest, preferably in the method according to an embodiement described herein.

A composition is disclosed for vacuum or inlet ionization comprising an ionization matrix, wherein the ionization matrix comprises or consists of salsalate.

Salsalate may have the following formula:

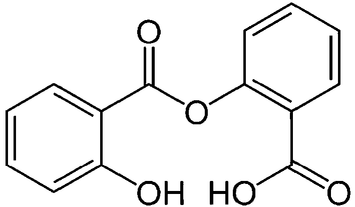

The composition may further comprise the at least one analyte.

The composition may further comprise a microparticle, preferable a magnetic particle, which is coated, wherein the coating may be a glass coating or a polymer coating.

The composition may further comprise a microparticle, preferable an immunobead.

The composition may be used for matrix-assisted ionization (MAI).

The molar ratio of the ionization matrix and analyte may range from 5:1 to 1x10⁷:1.

The composition may comprise a matrix:analyte-microparticle sample or a matrix:analyte sample, wherein the ionization matrix:analyte sample or the matrix:analyte-microparticle sample may be in asolid phase when exposed to sub-atmospheric pressure.

The ionization matrix may be crystallized when performing vacuum or inlet ionization.

The ionization matrix:analyte-microparticle sample or the matrix:analyte sample may be placed on a substrate, preferably as a spot.

The substrate may be selected form the group consisting of metal, paper, cloth, ribbon, glass, plastic, polymer, sodium dodecyl sulfate gel, agarose gel, paper chromatography plate, silica plate or woven fiber.

The composition may comprise a solvent.

The solvent may be water, methanol, ethanol, isopropanol, acetonitrile, tetrahydrofuran, chloroform, dimethylformamide, dimethyl sulfoxide, acetone, or mixtures thereof.

The matrix:analyte-microparticle sample or the matrix:analyte sample may be prepared by mixing or grinding the analyte and ionization matrix and optional the microparticle together.

The matrix:analyte-microparticle sample or matrix:analyte sample may be a solid.

The solid sample may be in a frozen state.

The matrix:analyte-microparticle sample or matrix:analyte sample may further comprise an ammonium salt, metal salt, acid, base, or buffer.

A Use of the composition of an embodiement described herein is disclosed for determining the at least one analyte of interest, preferably in the method of an embodiement described herein.

A kit suitable is disclosed to perform the method according to an embodiement described herein comprising
(A) an ionization matrix,
(B) a solvent or a further solvent,
(C) a microparticle, and
(D) optionally at least one internal standard.

A Use of the kit of an embodiement described herein in the method according to an embodiement described herein is also disclosed.

### Examples

The following examples are provided to illustrate, but not to limit the presently claimed invention.

### Example 1

As a first example a method for determining at least one analyte in a sample was conducted.

**Figure** 1 shows a schematic description of the method for determining at least one analyte in a sample, in particular the matrix ionization microparticle workflow. A model analyte, e.g. Leucine-Enkephalin, is pipetted to a horse serum matrix (e.g. 150 µl bulk volume). Accordingly, the model analyte is pipetted to a solution of H₂O/ACN (90/10, 150 µL bulk volume) for a blank measurement. Microparticles, e.g. magnetic bead particles, are added to the sample containing horse serum matrix and mixed properly. The analyte-microparticle complex is formed. After an incubation time of 10 min, the analyte-microparticle complex is washed two times with a solvent, e.g. water. Accordingly, an amount of the residual analyte-microparticle complex, e.g. as a dispersion, is transferred to a glass plate and a solution of the ionization matrix (e.g. 3-nitrobenzonitrile, 3-NBN) is added. Subsequently, at least the analyte and/or the mixture of ionization matrix and analyte-microparticle complex is measured by MS.

### Example 2

As a second example a method for determining at least one analyte in a sample was conducted.

**Figure** 2 shows a schematic description of the method for determining the at least one analyte in a sample, in particular the matrix ionization microparticle workflow. A model analyte is pipetted to a horse serum matrix (e.g. 150 µl bulk volume, H₂O/ACN=90/10). Accordingly, the model analyte is pipetted to a solution of H₂O/ACN (90/10, 150 µL bulk volume) for a blank measurement. Microparticles, e.g. magnetic bead particles, are added to the sample containing horse serum matrix and mixed properly. The analyte-microparticle complex is formed. After an incubation time of 10 min, the analyte-microparticle complex is washed two times with a solvent, e.g. water. Afterwards, an amount of an ionization matrix solution (e.g. 3-nitrobenzonitrile, 3-NBN) is pipetted to the washed analyte microparticle complex, e.g. as a dispersion. The ionization matrix solution and the analyte microparticle complex co-crystallize in the reaction vessel to form a matrix:analyte-microparticle sample. On the one hand, analyte molecules are extracted from the analyte microparticle complex and on the other hand, the analytes co-crystallize with the ionization matrix e.g. 3-NBN. The analyte is measured in the residual extraction liquid and in the co-crystallized matrix:analyte-microparticle sample or analyte-microparticle complex by MS.

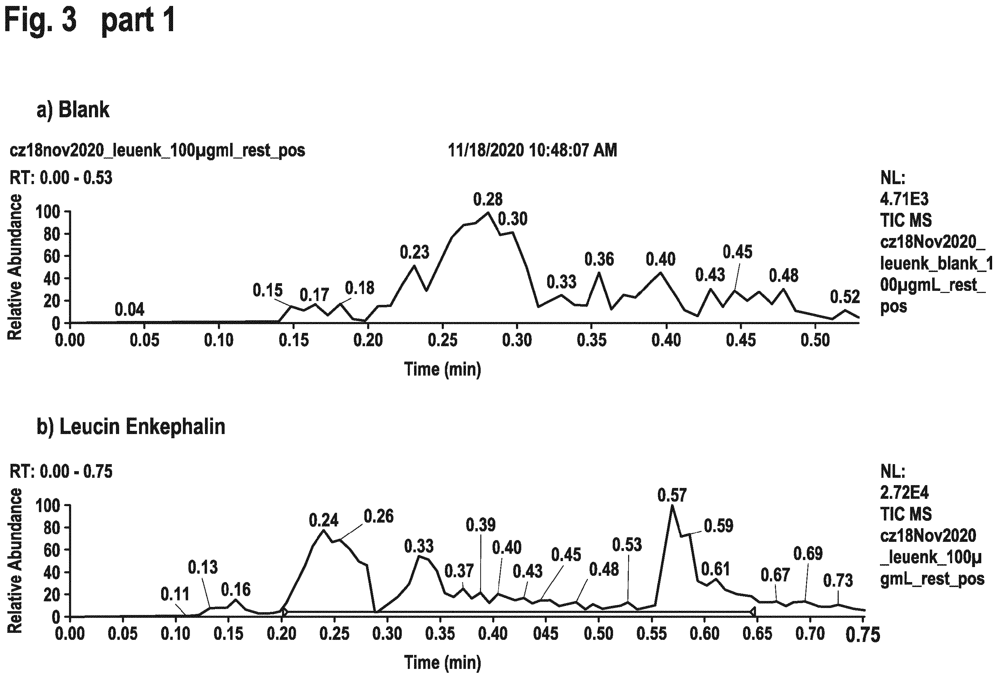

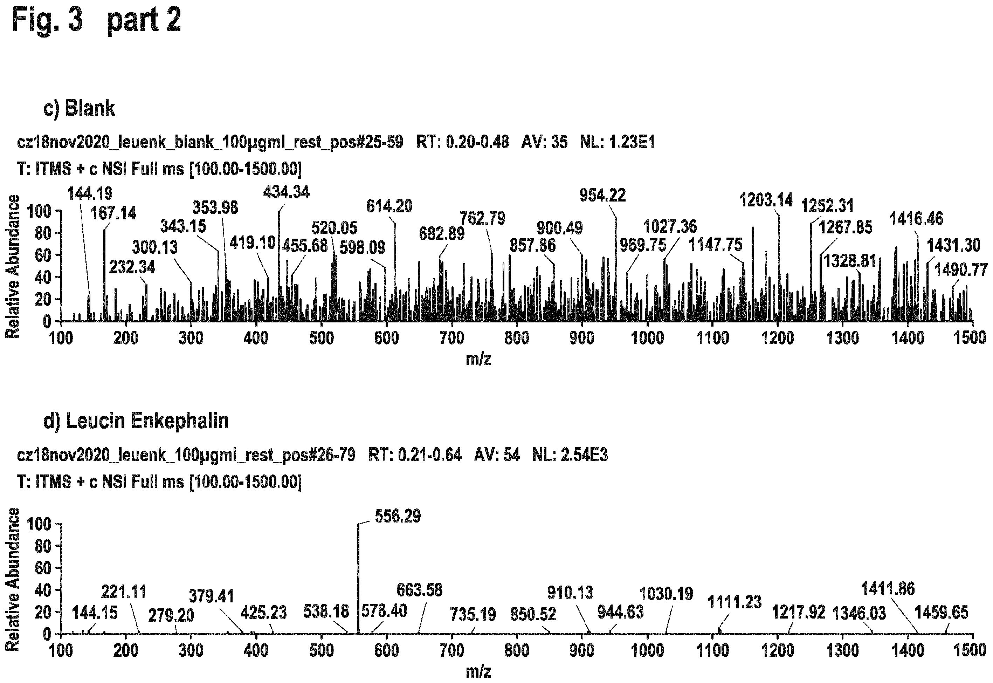

**Figures** 3 a) **to d)** show MS spectra (relative abundance vs. time and relative abundance vs. m/z, respectively) in positive ionization mode of 1 µL residual liquid after magnetic separation spotted on a glass plate. The relative abundances of the total ion current of the blank experiment (a) and analyte experiment (b) are shown. The corresponding mass spectra of the blank (c) shows various background signals at relatively low signal intensities. The analyte experiment (d) shows a distinct signal of Leucine-Enkaphalin at *m*/*z* 556. Therefore, by addition of the ionization matrix (e.g. 3-nitrobenzonitrile, 3-NBN), the model analyte showed a distinct MS signal without the use of further ionization energy. A sample without a spiked model analyte showed no signal after addition of the ionization matrix.

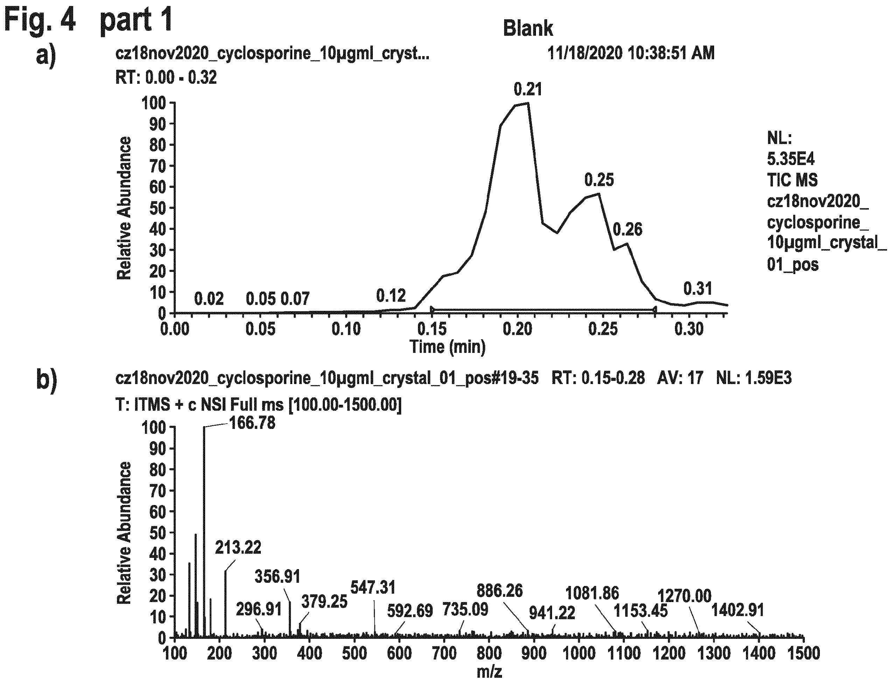

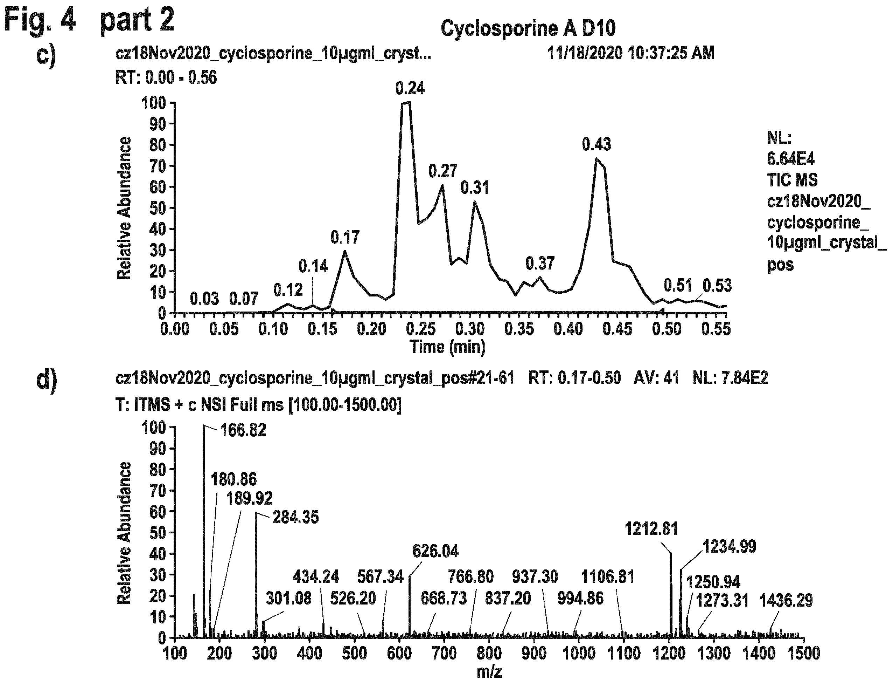

**Figures** 4 a) **to d)** show MS spectra of 1 µL of a mixture of recrystallized 3-NBN as the ionization matrix and analyte-loaded beads as the analyte-microparticle-complex. The spectrum of the blank experiment on the left side shows no Cyclosporine A D10 signals. The spectrum of the analyte experiment shows a signal at m/z 1213, corresponding to the [M+H]⁺ signal of Cyclosporine A D10. The analyte signal at m/z 1235 corresponds to the [M+Na]⁺ signal of Cyclosporine A D10. Magnetic microparticles were used for sample clean up and analyte/matrix separation. Crystallization of the analyte:microparticle complex, subsequent magnetic separation and measurement of the analyte/matrix mixture leads to distinct analyte signals.

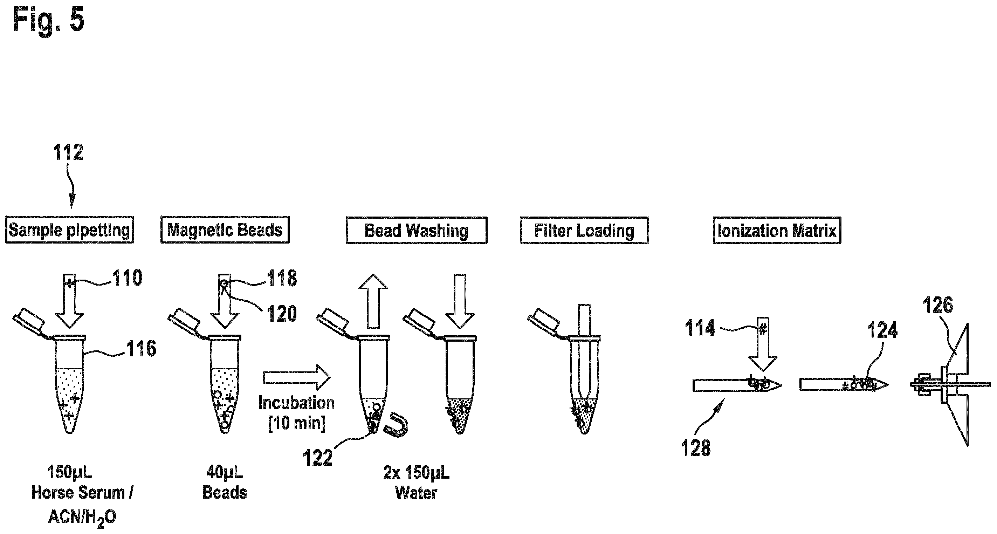

**Figure** 5 shows schematic description of the method for determining at least one analyte in a sample, in particular the matrix ionization microparticle workflow. A model analyte is pipetted to a horse serum matrix. Magnetic bead particles as microparticles are added to the sample containing horse serum matrix and mixed properly. After an incubation time of 10 min, the analyte-microparticle complex is washed two times with a solvent, e.g. water. After the last washing step, a triangle-shaped filter is placed into the residual analyte-microparticle complex (bead-analyte dispersion) and the analyte-microparticle complex is sucked into the filter tissue. Ionization matrix (e.g. 3-nitrobenzonitrile, 3-NBN, 100 mg/mL) is added to the filter tip and the mixture of ionization matrix and analyte-microparticle complex is measured by MS.

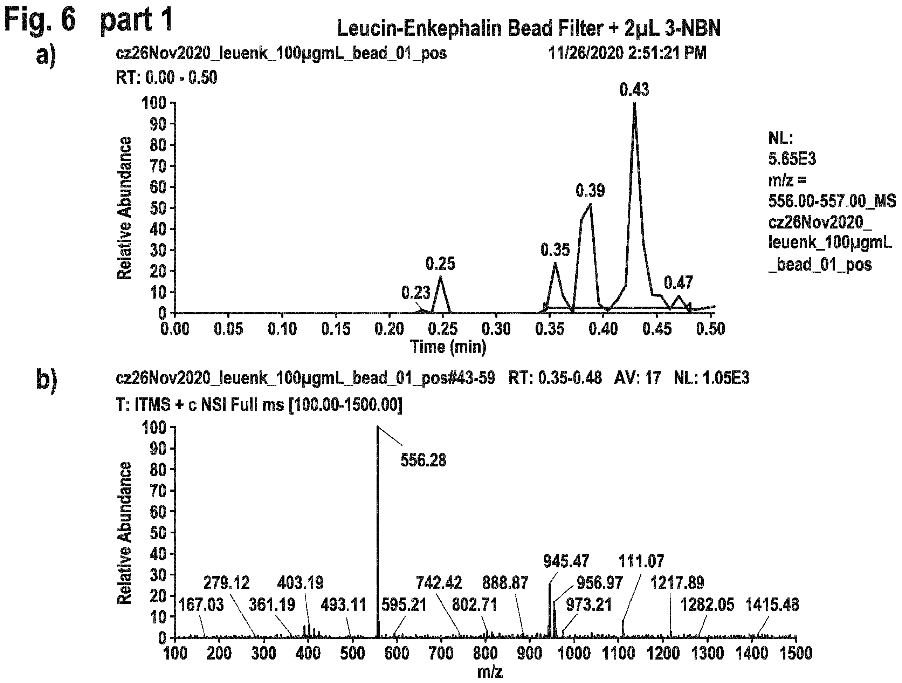

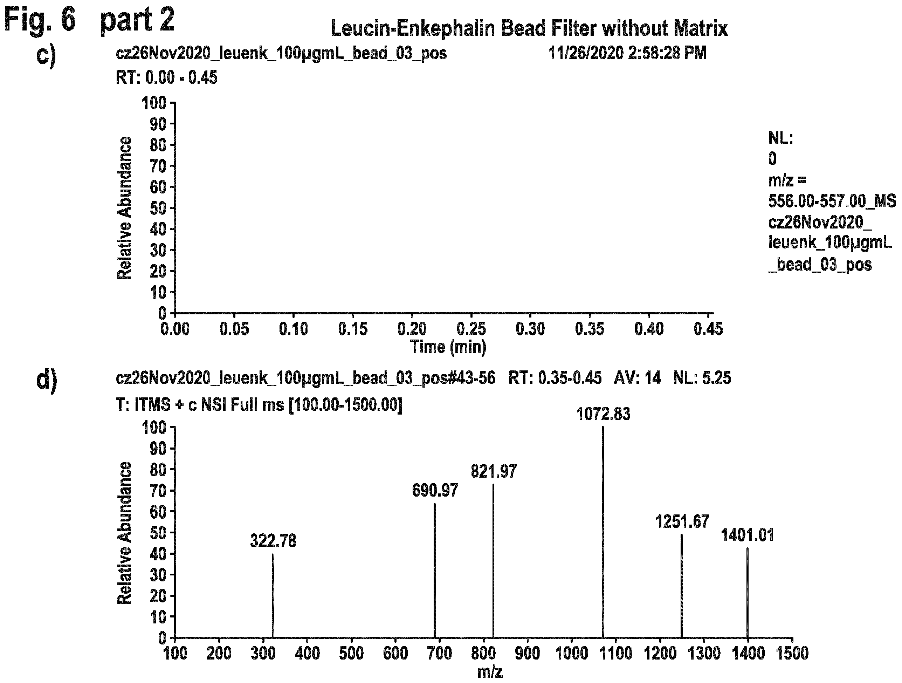

**Figures** 6 a) **to** d) show MS spectra of analyte-microparticle complex (bead-analyte dispersion) sucked into a triangle-shaped filter with addition of ionization matrix (a) and b)) and without ionization matrix (c) and d)). The spectrum of the analyte experiment on the left side shows an intense signal at m/z 556.3, corresponding to the [M+H]⁺ signal of Leucine-Enkephalin. The blank experiment on the right side shows no corresponding signals of Leucine-Enkephalin and nearly no background signals. A solid-phase microparticle sample extraction of an analyte and subsequent addition of an ionization matrix leads to distinct MS signals of the analyte. MS ionization is performed directly from the solid microparticle on a solid subtrate. A blank experiment without ionization matrix shows no analyte signals.

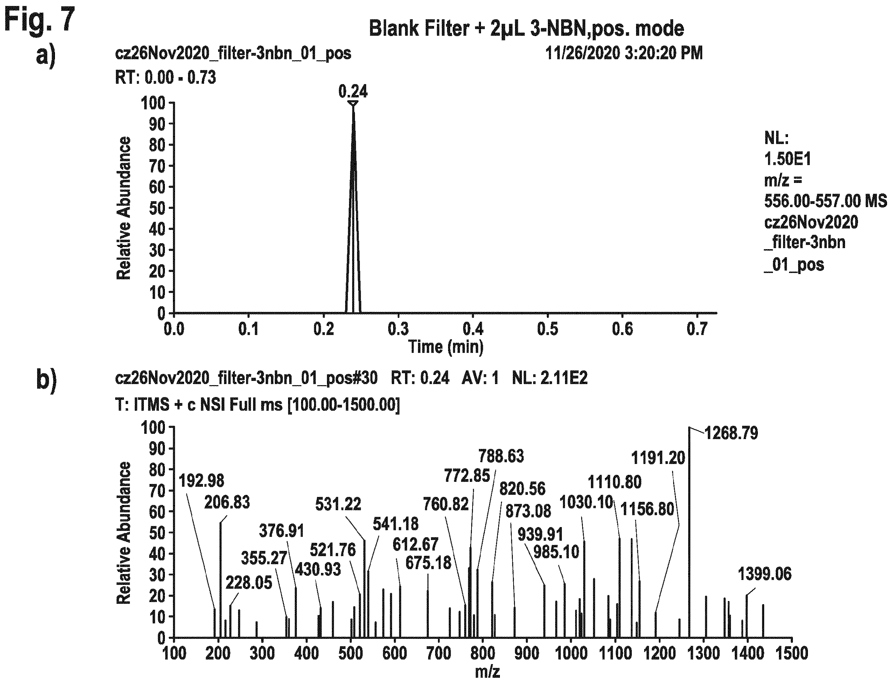

**Figures** 7 a) **and** b) show MS spectra of triangle-shaped filter with addition of ionization matrix and missing analyte-microparticle complex (bead-analyte dispersion). The blank experiment without analyte-microparticle complex (bead-analyte dispersion) shows no corresponding signals of Leucine-Enkephalin and nearly no background signals. Both blank experiments (no analyte (Fig. 6c and 6d) and no ionization matrix (Fig. 7a and 7b)) showed no background analyte signals and extremely low background noise.

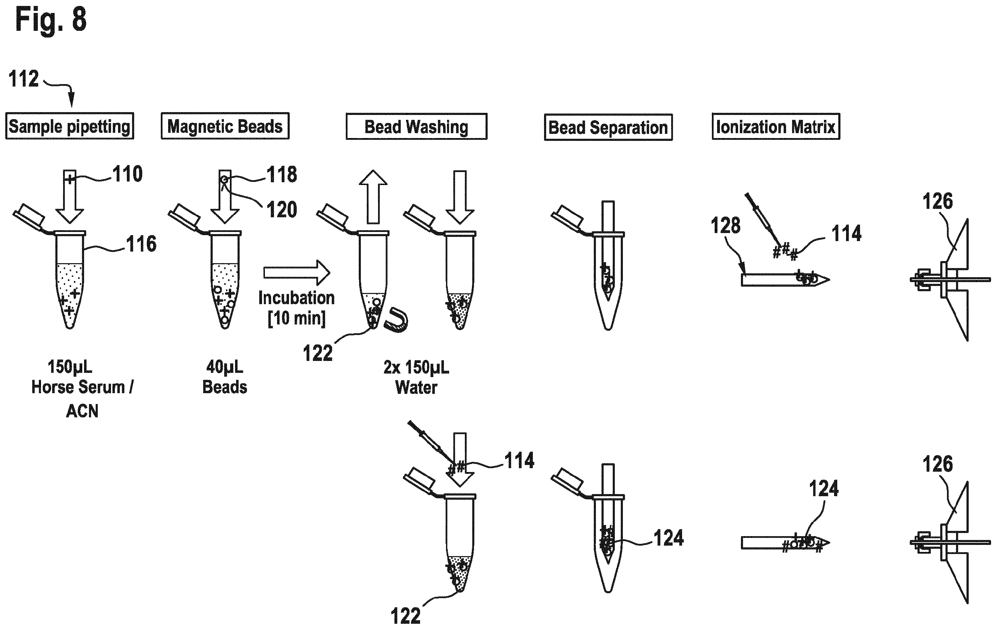

**Figure** 8 shows schematic descriptions of the method for determining at least one analyte in a sample, in particular the matrix ionization microparticle workflow. A model analyte is pipetted to a horse serum matrix. Magnetic bead particles as microparticles are added to the sample containing horse serum matrix and mixed properly. After an incubation time of 10 min, the analyte-microparticle complex is washed with a solvent, e.g. water.

In the first embodiment, after the last washing step, a triangle-shaped filter is placed into the residual analyte-microparticle complex (bead-analyte dispersion) and the analyte-microparticle complex is sucked into the filter tissue. Then the ionization matrix (e.g. 3-nitrobenzonitrile, 3-NBN, 100 mg/mL) is added to the filter tip and the mixture of ionization matrix and analyte-microparticle complex is measured by MS.

In the second embodiment after the last washing step, the ionization matrix is pipetted to the washed analyte-microparticle complex and a triangle-shaped filter is placed into the matrix:analyte-microparticle sample comprising the analyte-microparticle complex and the ionization matrix and the matrix:analyte-microparticle sample is sucked into the filter tissue. Then the mixture of ionization matrix and analyte-microparticle complex is measured by MS.

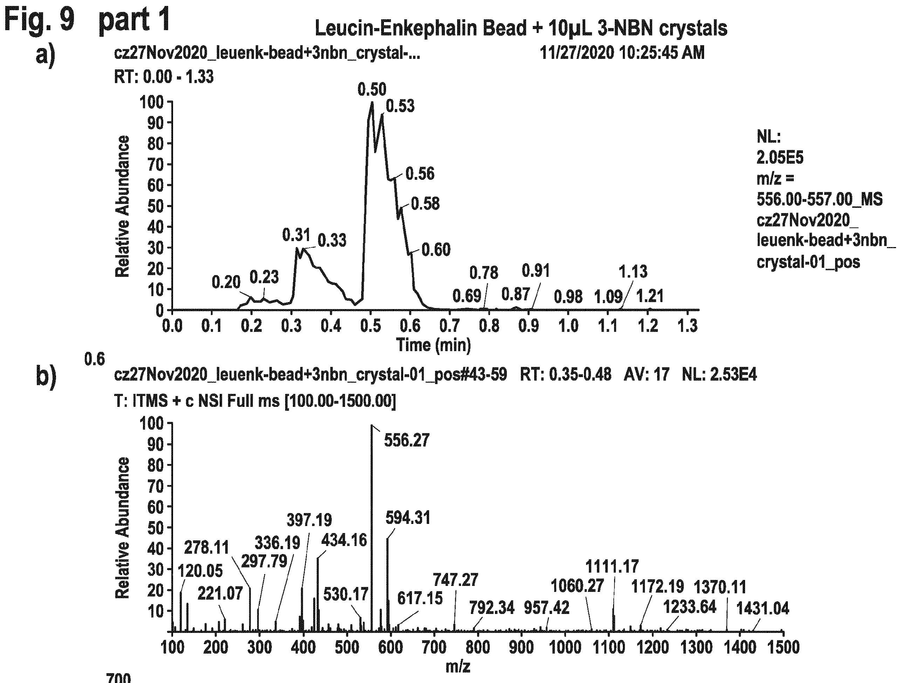

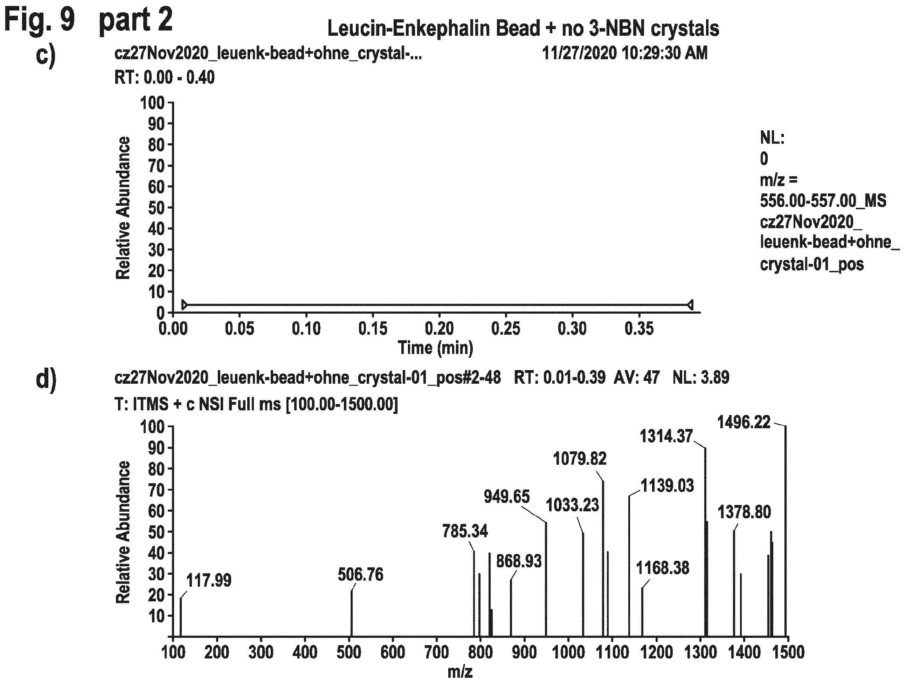

**Figures** 9 a) **to** d) show MS spectra of Leucine-Enkephalin coated microparticles with and without pre-crystallized ionization matrix, e.g. 3-NBN matrix. Crystallization of ionization matrix, e.g. 3-NBN was performed by mixing 20 µL of the ionization matrix e.g. 3-NBN (100 mg/mL in ACN+0.1% formic acid) + 10 µL H₂O. An aliquot of 10 µL of the pre-crystallized ionization matrix e.g. 3-NBN was first transferred to the washed analyte microparticle complex and loaded on the triangular-shaped filter. The spectrum of the analyte experiment on the left side shows an intense signal at m/z 556.3, corresponding to the [M+H]⁺ signal of Leucine-Enkephalin. The blank experiment on the right side shows no corresponding signals of Leucine-Enkephalin and nearly no background signals

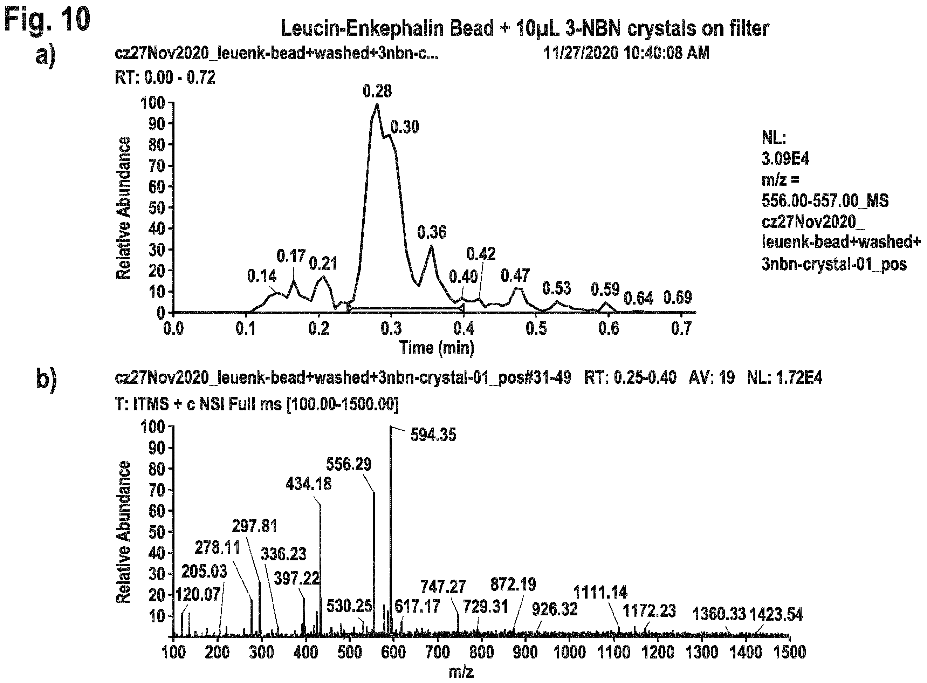

**Figures 10 a)** **and** b) show MS spectra of Leucine-Enkephalin coated microparticles with pre-crystallized ionization matrix, here 3-NBN. Crystallization of 3-NBN was performed by mixing 20 µL of 3-NBN (100 mg/mL in ACN+0.1% formic acid) + 10 µL H₂O. An aliquot of 10 µL of the pre-crystallized 3-NBN matrix was transferred to the analyte-loaded triangular-shaped filter. The spectrum of the analyte experiment shows a signal at m/z 556.3, corresponding to the [M+H]⁺ signal of Leucine-Enkephalin.

The Figures 9 and 10 showed, that the ionization matrix can either be pipetted directly onto the analyte-microparticle complex located on a substrate or added to the washed microparticle dispersion and subsequently loaded onto a substrate. Both pathways are a possible workflow. The corresponding blank experiment without ionization matrix showed no background and analyte signals.

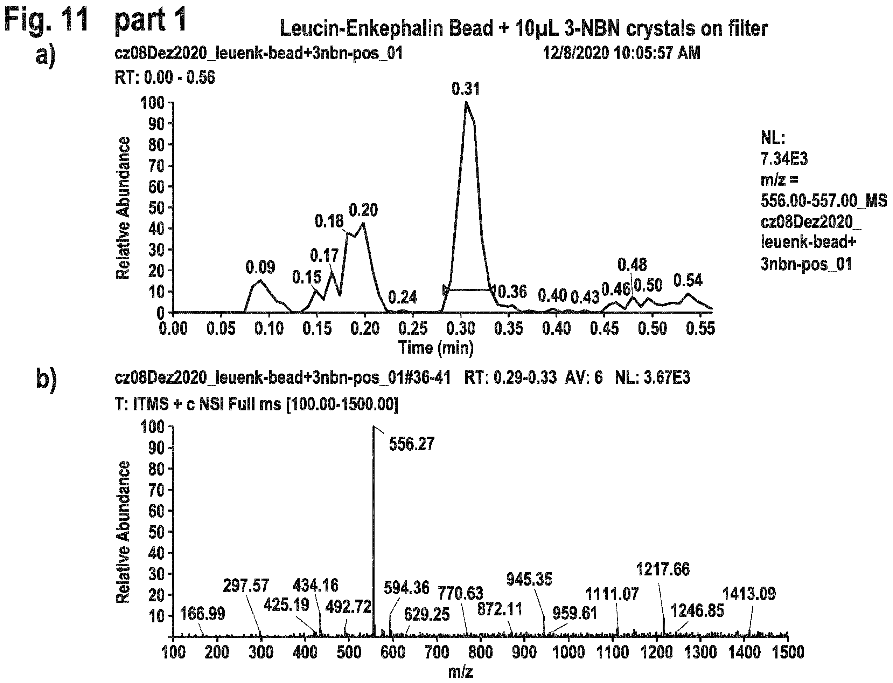

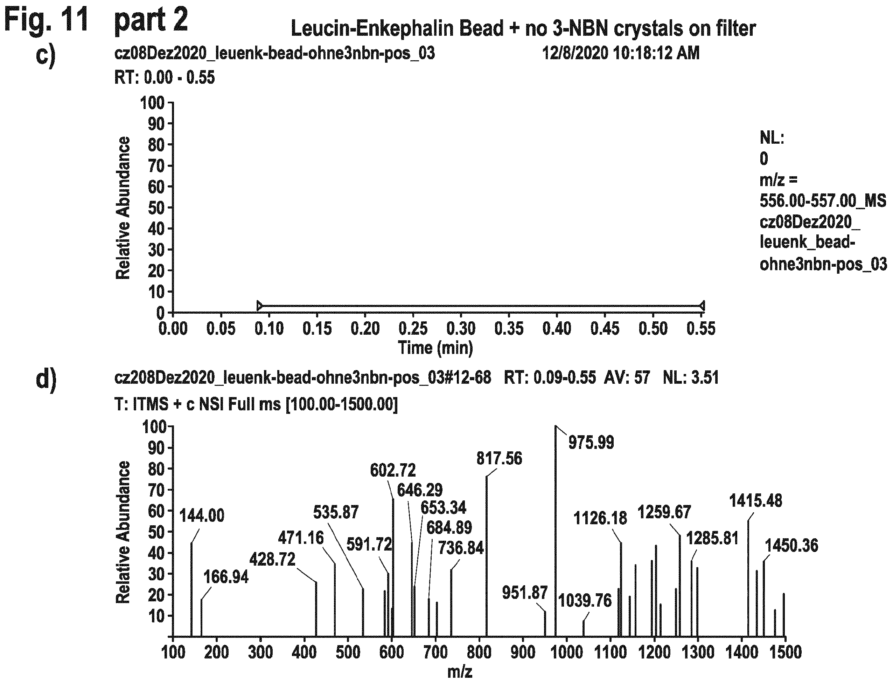

**Figures** 11 a) **to d)** show MS spectra of Leucine-Enkephalin coated microparticles with pre-crystallized ionization matrix, here 3-NBN. Crystallization of 3-NBN was performed by mixing 20 µL of 3-NBN (100 mg/mL in ACN+0.1% formic acid) + 10 µL H₂O. An aliquot of 10 µL of the pre-crystallized 3-NBN matrix was transferred to the analyte-loaded triangular-shaped filter. The spectrum of the analyte experiment on the left shows a signal at *m*/*z* 556.3, corresponding to the [M+H]⁺ signal of Leucine-Enkephalin. The blank experiment without 3-NBN matrix on the right shows no corresponding signals of Leucine-Enkephalin and nearly no background signals.

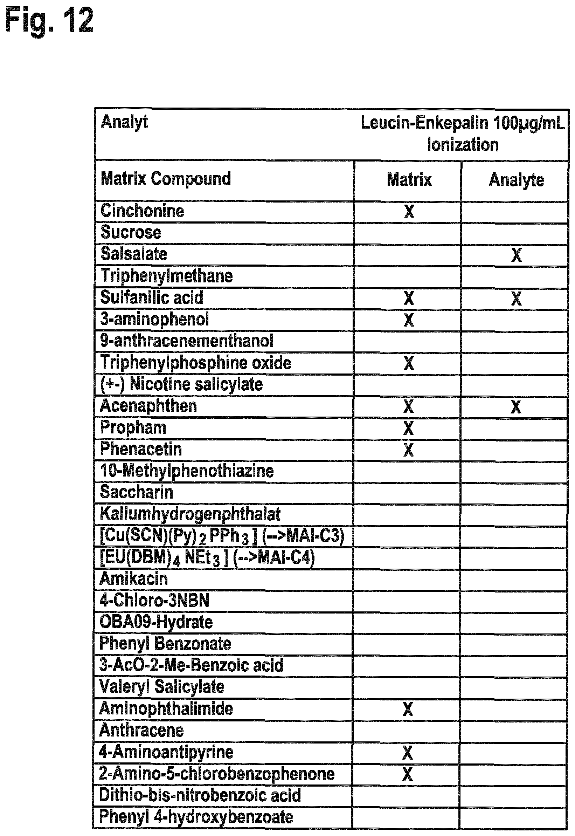

**Figure 12** shows a screening of different substances as ionization matrices, including the ionization matrix of salsalate. Leucine-Enkephalin was used as a model analyte in a concentration of 100 µg/mL. The ionization matrix and an analyte solution were mixed and directly measured by MS. The markings (X) stand for an MS signal of the matrix and/or the analyte alone. This means that only the ionization matrix of salsalate shows an analytes MS signal without in interfering the MS signal itself.

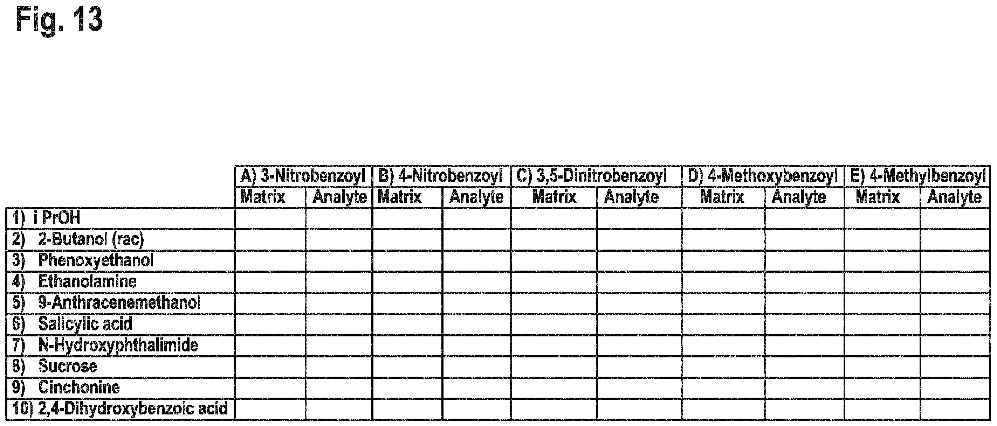

**Figure** 13 shows a screening of different nitrobenzene reaction products as ionization matrices. The nitrobenzene reaction products were previously prepared by a condensation reaction of the corresponding acid chlorides of structures A)-E) together with the molecules 1)-10). As ionization matrix, all nitrobenzene reaction products were dissolved (100 mg/mL in ACN+0.1% formic acid). Leucine-Enkephalin was used as a model analyte in a concentration of 100 µg/mL. The model analyte solution (1 µl) was mixed together with each ionization matrix solution (2 yL), co-crystallized and measured by MS. No MS signal of the matrix and/or the analyte was visible.

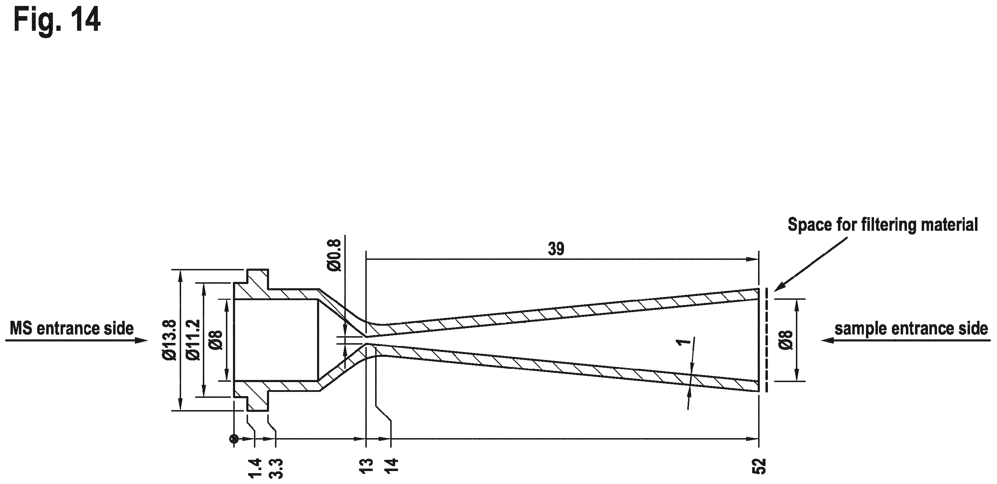

**Figure 14** shows an inlet for ion transport into the mass spectrometer. The inlet comprises a truncated sample entrance and a filter. The filter is arranged at the sample entrance of the conical, truncated inlet device for preventing the microparticle from entering the ion mobility spectrometry or mass spectrometry. The analyte-microparticle-complex according to step d) is held in front of the filter and truncated inlet device. The filter forms a barrier. The filter can be a nylon mesh, a membrane or a metal grid. The filter can be exchangeable. Other materials for the filter are possible, e.g. polyester mesh, poly(tetrafluoroethylene) filter membrane, polypropylene filter membrane or poly(ether ether ketone) filter membrane. The dimensions of the inlet as shown in Figure 14 are examples and can be varied.

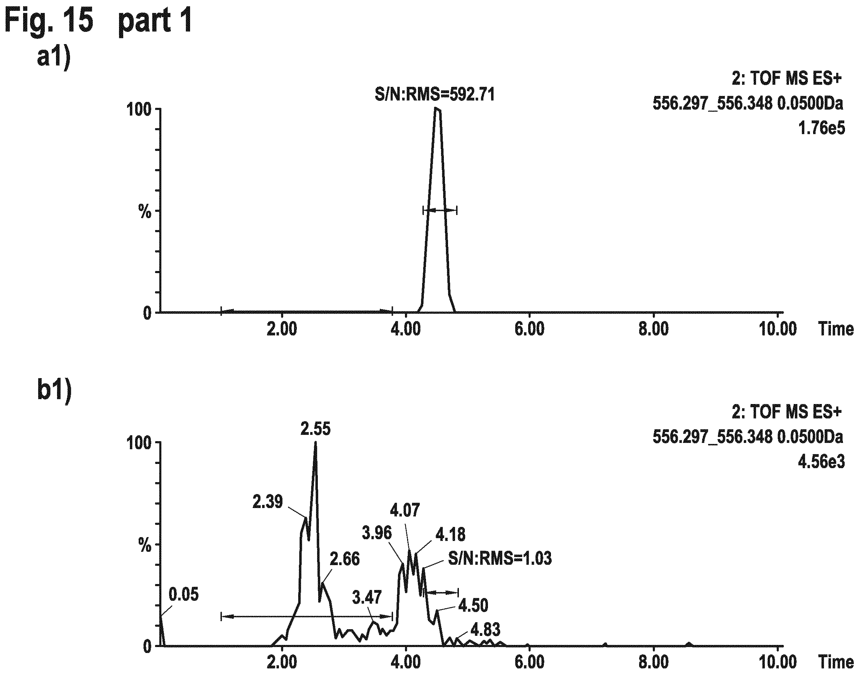

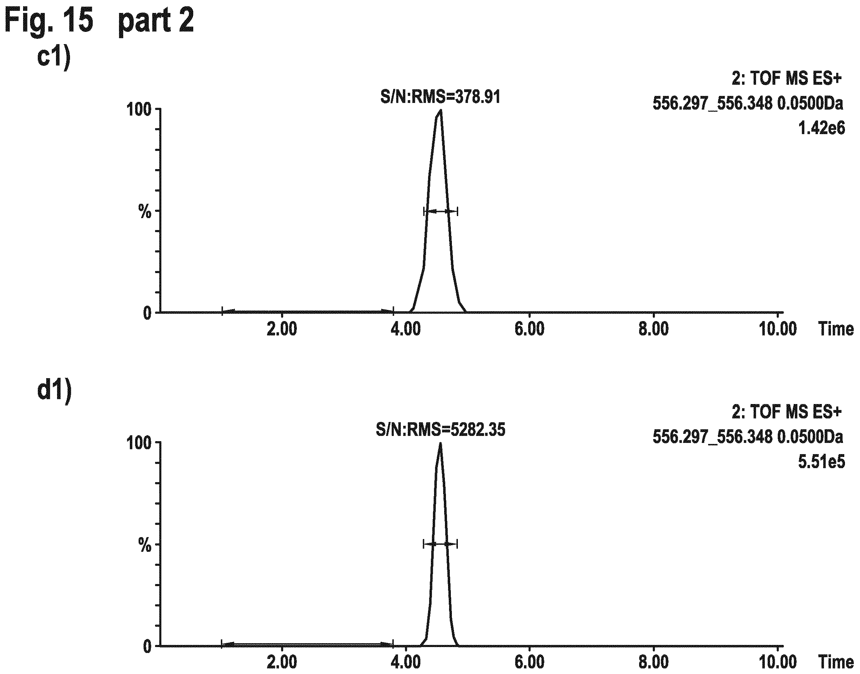

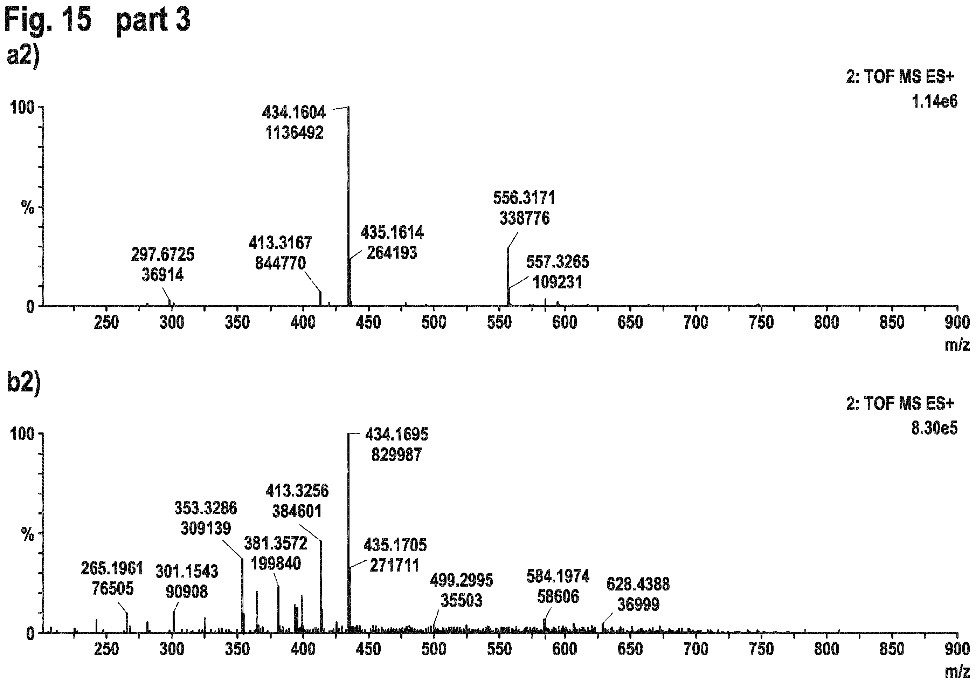

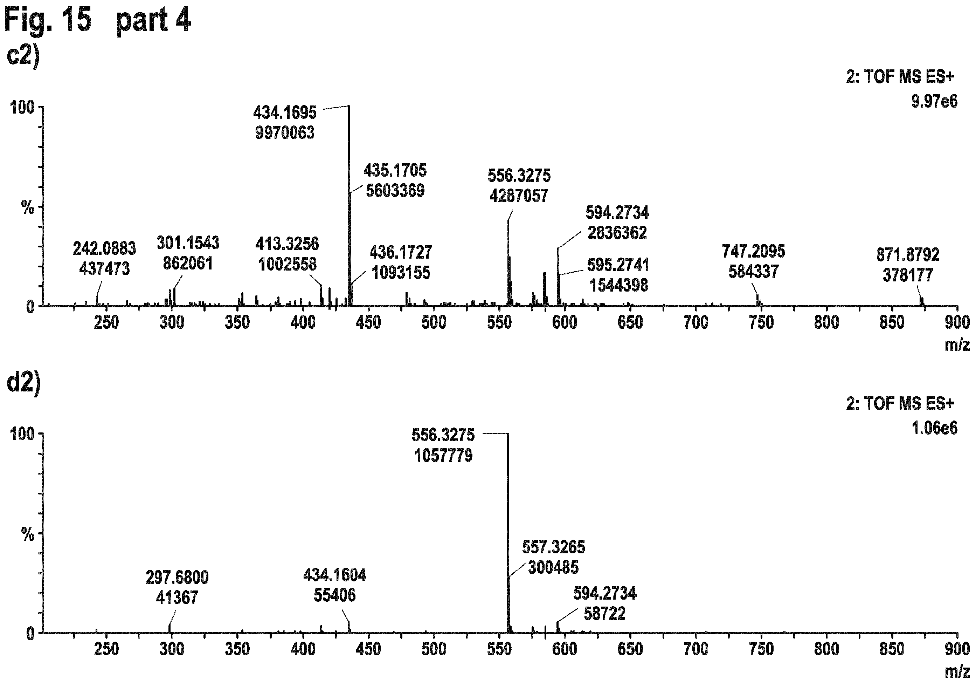

**Figures 15** **a1) to d2)** show respective extracted ion mobilograms (Fig. 15 a1)-15 d1); drift time range 0 ms to 10 ms) as well as the corresponding full scan mass spectra (Fig. 15a2)-15d2); *m*/*z* range 200 to 900) applying the inlet for ion transport into the mass spectrometer with or without the filtering material.

Figures 15d1) and 15d2) were recorded analyzing a crystallized spot of 1 µL Leucine-Enkephalin coated microparticle suspension (obtained from 100 µL of a 1 µg/mL aqueous solution) with 2 µL 3-NBN matrix (100 mg/mL in ACN+0.1% formic acid) in combination with a filtering material (woven nylon filter, 5 µm mesh size, Repligen). Figures 15c1) and 15c2) were recorded analyzing a crystallized spot of 1 µL Leucine-Enkephalin (1 µg/mL) coated microparticle suspension with 2 µL 3-NBN matrix (100 mg/mL in ACN+0.1% formic acid) without a filtering material. For comparison, Figures 15b1) and 15b2) were recorded analyzing solely a crystallized spot of 3-NBN matrix applying the inlet without a filtering material. Additionally, Figures 15a1) and 15b1) were recorded analyzing a crystallized spot of Leucine-Enkephalin (1 µL of an 1 µg/mL ageous solution) with 3-NBN matrix (2 µL, 100 mg/mL in ACN+0.1% formic acid). All spectra were recorded in IMS-ToF mode on a Synapt G2Si mass spectrometer (Waters) modified with the inlet for ion transport shown in Figure 14. The source temperature was set to 50 °C and every crystallized sample was measured for a total of 30 s analysis time. Nitrogen was used as IMS drift gas. The settings of the IMS wave height and the wave velocity were 30 V, respectively 800 m/s. The extracted ion mobilograms were obtained by extracting the Leucine-Enkephalin [M+H]⁺ signal at *m*/*z* 556.3. For determining the S/N ratio, the signal range was set between 4.2 ms and 4.8 ms, whereas the noise range was set between 1.0 ms and 3.8 ms. The number of counts observed from the Leucine-Enkephalin coated microparticles in Figure 15c1) clearly increased compared to the Leucin-Enkephalin solution itself (Fig. 15a1)). The blank 3-NBN matrix in Figure 15b1) itself shows no detection of Leucine-Enkephalin [M+H]⁺ signal, but a certain noise level. Applying the filtering material between the sample and the inlet resulting in significant reduction of background signals in the mass spectrum of Figure 15d2) compared to Figure 15c2), while significantly increasing the S/N ratio in the extracted ion mobilogram of Figure 15d1) compared to Figure 15c1).

### List of reference numbers

- 110: analyte
- 112: sample
- 114: ionization matrix
- 116: vessel
- 118: microparticle
- 120: surface of the microparticle
- 122: analyte-microparticle-complex
- 124: matrix:analyte-microparticle sample
- 126: ion mobility spectrometry and/or mass spectrometry
- 128: substrate surface

## Claims

1. A method for determining at least one analyte (110) in a sample (112), wherein the method comprises the following steps:
a) Providing the at least one analyte (110), at least one microparticle (118), at least one ionization matrix (114), and a substrate having a substrate surface (128),
b) Incubating the analyte (110) with the microparticle (118) having at least one microparticle surface (120), wherein the analyte is adsorbed on the surface of the microparticle (120) and an analyte-microparticle-complex (122) is formed, wherein the ionization matrix (114) and/or analyte-microparticle-complex (122) are crystallized,
c) Contacting the analyte-microparticle-complex (122) with the ionization matrix (114) to form a matrix:analyte-microparticle sample (124),
d) Providing the matrix:analyte-microparticle sample (124) on the substrate surface (128),
e) Ionization at least the analyte (110), wherein the ionization is a mechanical ionization,
f) Determining the analyte (110) via ion mobility spectrometry and/or mass spectrometry (126),
wherein the mechanical ionization is induced by a mechanical force, which comprises or consists of a shear force and/or
wherein the mechanical ionization is induced by mechanical stimulation, wherein the mechanical stimulation is triboluminescence.

2. The method according to claim 1, wherein the ionization in step e) is a matrix assisted ionization (MAI), preferably a double solid supported matrix assisted ionization.

3. The method according to any of the proceeding claims,
wherein the microparticle (118) is a magnetic particle or an immunobead,
wherein the microparticle (118) is coated with a glass coating or a polymer coating, if the microparticle (118) is a magnetic particle.

4. The method according to any of the proceeding claims, wherein the ionization matrix (114) is selected from the group consisting of salsalate, 3-nitrobenzonitrile, 2,2'-azobis(2-methylpropane), 2-nitrobenzonitrile, 5-methyl-2-nitrobenzonitrile, coumarin, methyl-2-methyl-3-nitrobenzoate, methyl-5-nitro-2-furoate, 2-bromo-2-nitropropane-1,3-diol, 3-nitrobenzaldehyde, 6-nitro-o-anisonitrile, phthalic anhydride, or mixtures thereof.

5. A system automated to perfom the method of any one of claims 1 to 4, the system comprising:
a sample element for determining at least one analyte and for performing the method according to any of the proceeding claims 1 to 4, the sample element comprising:
- a substrate surface (128),
- an ionization matrix (114) arranged on the substrate surface (128) and for using matrix-assisted ionization,
- an analyte-microparticle-complex (122) arranged on the substrate surface (128),
- wherein the ionization matrix (114) is selected from the group consisting of salsalate, 3-nitrobenzonitrile, 2,2'-azobis(2-methylpropane), 2-nitrobenzonitrile, 5-methyl-2- nitrobenzonitrile, coumarin, methyl-2-methyl-3-nitrobenzoate, methyl-5-nitro-2-furoate, 2-bromo-2-nitropropane-1,3-diol, 3-nitrobenzaldehyde, 6-nitro-o-anisonitrile, phthalic anhydride, or mixtures thereof,
- wherein the ionization matrix (114) and/or analyte-microparticle-complex (122) are crystallized and configured to exhibit a mechanical force caused by shear force and/or triboluminescence for mechanical ionization of the analyte,
- wherein the microparticle (118) of the analyte-microparticle-complex (122) is magnetic,
- wherein the analyte-microparticle-complex (122) and ionization matrix (114) are in contact to each other; and
the system further comprising:
an inlet for performing the method according to any of the proceeding claims 1 to 4 and for ion transport into the mass spectrometer or ion mobility spectrometer (126) or into the detector of the mass spectrometer or ion mobility spectrometer comprising a truncated sample entrance and a filter.

## Patentansprüche

1. Verfahren zur Bestimmung von mindestens einem Analyten (110) in einer Probe (112), wobei das Verfahren die folgenden Schritte umfasst:
a) Bereitstellen des mindestens einen Analyten (110), mindestens eines Mikropartikels (118), mindestens einer Ionisierungsmatrix (114) und eines Substrats mit einer Substratoberfläche (128),
b) Inkubieren des Analyten (110) mit dem Mikropartikel (118) mit mindestens einer Mikropartikeloberfläche (120), wobei der Analyt an der Oberfläche des Mikropartikels (120) adsorbiert wird und ein Analyt-Mikropartikel-Komplex (122) gebildet wird, wobei die Ionisierungsmatrix (114) und/oder der Analyt-Mikropartikel-Komplex (122) kristallisiert werden,
c) Inkontaktbringen des Analyt-Mikropartikel-Komplexes (122) mit der Ionisierungsmatrix (114) unter Bildung einer Matrix:Analyt-Mikropartikel-Probe (124),
d) Bereitstellen der Matrix:Analyt-Mikropartikel-Probe (124) auf der Substratoberfläche (128),
e) Ionisieren mindestens des Analyten (110), wobei die Ionisierung eine mechanische Ionisierung ist,
f) Bestimmen des Analyten (110) über Ionenmobilitätsspektrometrie und/oder Massenspektrometrie (126),
wobei die mechanische Ionisierung durch eine mechanische Kraft induziert wird, die eine Scherkraft umfasst oder daraus besteht, und/oder
wobei die mechanische Ionisierung durch mechanische Stimulation induziert wird, wobei die mechanische Stimulation Tribolumineszenz ist.

2. Verfahren nach Anspruch 1, wobei die Ionisierung in Schritt e) eine matrixgestützte Ionisierung (MAI), vorzugsweise eine doppelfestkörpergestützte matrixgestützte Ionisierung, ist.

3. Verfahren nach einem der vorhergehenden Ansprüche,
wobei das Mikropartikel (118) ein magnetisches Partikel oder ein Immunkügelchen ist,
wobei das Mikropartikel (118) mit einer Glasbeschichtung oder einer Polymerbeschichtung beschichtet ist, wenn das Mikropartikel (118) ein magnetisches Partikel ist.

4. Verfahren nach einem der vorhergehenden Ansprüche, wobei die Ionisierungsmatrix (114) ausgewählt ist aus der Gruppe, bestehend aus Salsalat, 3-Nitrobenzonitril, 2,2'-Azobis(2-methylpropan), 2-Nitrobenzonitril, 5-Methyl-2-nitrobenzonitril, Cumarin, Methyl-2-methyl-3-nitrobenzoat, Methyl-5-nitro-2-furoat, 2-Brom-2-nitropropan-1,3-diol, 3-Nitrobenzaldehyd, 6-Nitro-o-anisonitril, Phthalsäureanhydrid oder Mischungen davon.

5. Automatisiertes System zur Durchführung des Verfahrens nach einem der Ansprüche 1 bis 4, wobei das System Folgendes umfasst:
ein Probenelement zur Bestimmung von mindestens einem Analyten und zur Durchführung des Verfahrens nach einem der vorhergehenden Ansprüche 1 bis 4, wobei das Probenelement Folgendes umfasst:
- eine Substratoberfläche (128),
- eine Ionisierungsmatrix (114), die auf der Substratoberfläche (128) angeordnet ist und zur Verwendung von matrixgestützter Ionisierung dient,
- einen Analyt-Mikropartikel-Komplex (122), der auf der Substratoberfläche (128) angeordnet ist,
- wobei die Ionisierungsmatrix (114) ausgewählt ist aus der Gruppe, bestehend aus Salsalat, 3-Nitrobenzonitril, 2,2'-Azobis(2-methylpropan), 2-Nitrobenzonitril, 5-Methyl-2-nitrobenzonitril, Cumarin, Methyl-2-methyl-3-nitrobenzoat, Methyl-5-nitro-2-furoat, 2-Brom-2-nitropropan-1,3-diol, 3-Nitrobenzaldehyd, 6-Nitro-o-anisonitril, Phthalsäureanhydrid oder Mischungen davon,
- wobei die Ionisierungsmatrix (114) und/oder der Analyt-Mikropartikel-Komplex (122) kristallisiert werden und so konfiguriert sind, dass sie eine mechanische Kraft, die durch Scherkraft und/oder Tribolumineszenz verursacht wird, zur mechanischen Ionisierung des Analyten aufweisen,
- wobei das Mikropartikel (118) des Analyt-Mikropartikel-Komplexes (122) magnetisch ist,
- wobei der Analyt-Mikropartikel-Komplex (122) und die Ionisierungsmatrix (114) miteinander in Kontakt stehen; und
wobei das System ferner Folgendes umfasst:
einen Einlass zur Durchführung des Verfahrens nach einem der vorhergehenden Ansprüche 1 bis 4 und zum Ionentransport in das Massenspektrometer oder Ionenmobilitätsspektrometer (126) oder in den Detektor des Massenspektrometers oder Ionenmobilitätsspektrometers, umfassend einen verkürzten Probeneingang und einen Filter.

## Revendications

1. Procédé de détermination d'au moins une substance à analyser (110) dans un échantillon (112), dans lequel le procédé comprend les étapes suivantes :
a) fourniture de l'au moins une substance à analyser (110), d'au moins une microparticule (118), d'au moins une matrice d'ionisation (114) et d'un substrat ayant une surface de substrat (128),
b) incubation de la substance à analyser (110) avec la microparticule (118) ayant au moins une surface de microparticule (120), dans lequel la substance à analyser est adsorbée sur la surface de la microparticule (120) et un complexe substance à analyser-microparticule (122) est formé, dans lequel la matrice d'ionisation (114) et/ou le complexe substance à analyser-microparticule (122) sont cristallisés,
c) mise en contact du complexe substance à analyser-microparticule (122) avec la matrice d'ionisation (114) pour former un échantillon matrice:substance à analyser-microparticule (124),
d) fourniture de l'échantillon matrice:substance à analyser-microparticule (124) à la surface du substrat (128),
e) ionisation d'au moins la substance à analyser (110), dans lequel l'ionisation est une ionisation mécanique,
f) détermination de la substance à analyser (110) par l'intermédiaire d'une spectrométrie de mobilité ionique et/ou d'une spectrométrie de masse (126),
dans lequel l'ionisation mécanique est induite par une force mécanique, qui comprend ou consiste en une force de cisaillement et/ou
dans lequel l'ionisation mécanique est induite par stimulation mécanique, dans lequel la stimulation mécanique est la triboluminescence.

2. Procédé selon la revendication 1, dans lequel l'ionisation à l'étape e) est une ionisation assistée par matrice (MAI), de préférence une ionisation assistée par matrice sur double support solide.

3. Procédé selon l'une quelconque des revendications précédentes,
dans lequel la microparticule (118) est une particule magnétique ou une immunobille,
dans lequel la microparticule (118) est revêtue d'un revêtement de verre ou d'un revêtement polymère, si la microparticule (118) est une particule magnétique.

4. Procédé selon l'une quelconque des revendications précédentes, dans lequel la matrice d'ionisation (114) est choisie dans le groupe constitué par le salsalate, le 3-nitrobenzonitrile, le 2,2'-azobis(2-méthylpropane), le 2-nitrobenzonitrile, le 5-méthyl-2-nitrobenzonitrile, la coumarine, le 2-méthyl-3-nitrobenzoate de méthyle, le 5-nitro-2-furoate de méthyle, le 2-bromo-2-nitropropane-1,3-diol, le 3-nitrobenzaldéhyde, le 6-nitro-o-anisonitrile, l'anhydride phtalique, ou des mélanges de ceux-ci.

5. Système automatisé pour réaliser le procédé selon l'une quelconque des revendications 1 à 4, le système comprenant :
un élément d'échantillon pour déterminer au moins une substance à analyser et pour réaliser le procédé selon l'une quelconque des revendications 1 à 4 précédentes, l'élément d'échantillon comprenant :
- une surface de substrat (128),
- une matrice d'ionisation (114) agencée sur la surface du substrat (128) et pour utiliser une ionisation assistée par matrice,
- un complexe substance à analyser-microparticule (122) agencé sur la surface du substrat (128),
- dans lequel la matrice d'ionisation (114) est choisie dans le groupe constitué par le salsalate, le 3-nitrobenzonitrile, le 2,2'-azobis(2-méthylpropane), le 2-nitrobenzonitrile, le 5-méthyl-2-nitrobenzonitrile, la coumarine, le 2-méthyl-3-nitrobenzoate de méthyle, le 5-nitro-2-furoate de méthyle, le 2-bromo-2-nitropropane-1,3-diol), le 3-nitrobenzaldéhyde, le 6-nitro-o-anisonitrile, l'anhydride phtalique, ou des mélanges de ceux-ci,
- dans lequel la matrice d'ionisation (114) et/ou le complexe substance à analyser-microparticule (122) sont cristallisés et conçus pour présenter une force mécanique provoquée par une force de cisaillement et/ou une triboluminescence pour l'ionisation mécanique de la substance à analyser,
- dans lequel la microparticule (118) du complexe substance à analyser-microparticule (122) est magnétique,
- dans lequel le complexe substance à analyser-microparticule (122) et la matrice d'ionisation (114) sont en contact l'une avec l'autre ; et
le système comprenant en outre :
une entrée pour réaliser le procédé selon l'une quelconque des revendications 1 à 4 précédentes et pour le transport des ions dans le spectromètre de masse ou le spectromètre de mobilité ionique (126) ou dans le détecteur du spectromètre de masse ou du spectromètre de mobilité ionique comprenant une entrée d'échantillon tronquée et un filtre.
